(19) Europäisches Patentamt
European Patent Office
Office européen des brevets

(11) EP 1 009 776 B1

(12) # EUROPÄISCHE PATENTSCHRIFT

(45) Veröffentlichungstag und Bekanntmachung des Hinweises auf die Patenterteilung:
**10.12.2003 Patentblatt 2003/50**

(21) Anmeldenummer: **98948893.7**

(22) Anmeldetag: **01.09.1998**

(51) Int Cl.7: **C09B 67/00**, C09K 19/00, C09D 5/36

(86) Internationale Anmeldenummer:
**PCT/EP98/05545**

(87) Internationale Veröffentlichungsnummer:
**WO 99/011719 (11.03.1999 Gazette 1999/10)**

(54) **MEHRSCHICHTIGE CHOLESTERISCHE PIGMENTE**

MULTILAYER CHOLESTERIC PIGMENTS

PIGMENTS CHOLESTERIQUES MULTICOUCHES

(84) Benannte Vertragsstaaten:
**CH DE FR GB IT LI NL**

(30) Priorität: **02.09.1997 DE 19738368**
**23.12.1997 DE 19757699**

(43) Veröffentlichungstag der Anmeldung:
**21.06.2000 Patentblatt 2000/25**

(73) Patentinhaber: **BASF AKTIENGESELLSCHAFT**
**67056 Ludwigshafen (DE)**

(72) Erfinder:
- **SCHUHMACHER, Peter**
  **D-68163 Mannheim (DE)**
- **SCHNEIDER, Norbert**
  **D-67122 Altrip (DE)**
- **RICHTER, Volker**
  **D-69118 Heidelberg (DE)**
- **KELLER, Harald**
  **D-67069 Ludwigshafen (DE)**
- **BLASCHKA, Peter**
  **D-67069 Ludwigshafen (DE)**
- **BETTINGER, Günter**
  **D-67105 Schifferstadt (DE)**
- **MEYER, Frank**
  **D-68165 Mannheim (DE)**
- **HEILMANN, Peter**
  **D-67098 Bad Dürkheim (DE)**
- **BEST, Wolfgang**
  **D-67251 Freinsheim (DE)**

(74) Vertreter: **Kinzebach, Werner, Dr. et al**
**Ludwigsplatz 4**
**67059 Ludwigshafen (DE)**

(56) Entgegenhaltungen:

| | |
|---|---|
| **EP-A- 0 872 336** | **DE-A- 19 619 973** |
| **GB-A- 2 276 883** | **GB-A- 2 282 145** |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 009 776 B1

Printed by Jouve, 75001 PARIS (FR)

## Beschreibung

Die Erfindung betrifft mehrschichtige cholesterische Pigmente, Verfahren zu ihrer Herstellung und ihre Verwendung.

Beim Erwärmen formanisotroper Stoffe können flüssigkristalline Phasen, sogenannte Mesophasen, auftreten. Die einzelnen Phasen unterscheiden sich durch die räumliche Anordnung der Molekülschwerpunkte einerseits sowie durch die Molekülanordnung hinsichtlich der Längsachsen andererseits (G. W. Gray, P. A. Winsor, Liquid Crystals and Plastic Crystals, Ellis Horwood Limited, Chichester 1974). Die nematisch flüssigkristalline Phase zeichnet sich durch Parallelorientierung der Molekül-Längsachsen aus (eindimensionaler Ordnungszustand). Unter der Voraussetzung, daß die die nematische Phase aufbauenden Moleküle chiral sind, entsteht eine sogenannte chiral nematische (cholesterische) Phase, bei der die Längsachsen der Moleküle eine zu ihr senkrechte, helixartige Überstruktur ausbilden (H. Baessler, Festkörperprobleme XI, 1971). Der chirale Molekülteil kann sowohl im flüssigkristallinen Molekül selbst vorhanden sein als auch als Dotierstoff zur nematischen Phase gegeben werden, wodurch die chiral nematische Phase induziert wird. Dieses Phänomen wurde zuerst an Cholesterolderivaten untersucht (z. B. H. Baessler, M. M. Labes, *J. Chem. Phys. 52*, 631 (1970)).

Die chiral nematische Phase hat besondere optische Eigenschaften: eine hohe optische Rotation sowie einen ausgeprägten Zirkulardichroismus, der durch Selektivreflektion von zirkular polarisiertem Licht innerhalb der chiral nematischen Schicht entsteht. In Abhängigkeit von der Ganghöhe der helixartigen Überstruktur können unterschiedliche Farben erzeugt werden, die je nach Blickwinkel unterschiedlich erscheinen können. Die Ganghöhe der helixartigen Überstruktur ist ihrerseits vom Verdrillungsvermögen der chiralen Komponente abhängig. Dabei kann insbesondere durch Änderung der Konzentration eines chiralen Dotierstoffs die Ganghöhe und damit der Wellenlängenbereich des selektiv reflektierten Lichts einer chiral nematischen Schicht variiert werden. Solche chiral nematischen Systeme bieten für eine praktische Anwendung interessante Möglichkeiten.

Cholesterische Effektpigmente und Zusammensetzungen, die solche Pigmente enthalten, sind bekannt.

Die EP-A-686 674 und die ihr zugrunde liegende DE-A-44 16 191 beschreibt Interferenzpigmente aus in cholesterischer Anordnung fixierten Molekülen; die Pigmente weisen eine plättchenförmige Struktur und eine, durch ein Beispiel belegte, Schichtdicke von 7 µm auf. Zur Herstellung der Pigmente wird hochviskoses (zähflüssiges) LC-Material auf einen Träger aufgebracht, wobei der Träger unter einer fixierten Rakel mit einer Laufgeschwindigkeit von etwa 2 m/min fortbewegt wird. Hierdurch werden die flüssigkristallinen Moleküle orientiert.

Die WO 97/30136 beschreibt cholesterische Polymerplättchen, die aus einem chiralen polymerisierbaren mesogenen Material erhältlich sind. Die Plättchen sind als Effektpigmente verwendbar. Sie sind einschichtig und weisen eine bevorzugte Dicke von 4 bis 10 µm auf.

Aus der DE-A-196 39 179 sind lichtdurchlässige Zusammensetzungen bekannt, die Pigmente mit vom Betrachtungswinkel abhängiger Farbigkeit enthalten. Die Zusammensetzungen sollen absorptive Farbmittel in einer Menge enthalten, die bewirkt, dass der bei Glanzwinkelkonfiguration auftretende winkelabhängige Farbeffekt verstärkt wird, ohne dass bei allen anderen Winkelkonfigurationen die Transparenz der Zusammensetzung entscheidend beeinträchtigt wird. Die Herstellung der Pigmente erfolgt, wie in der oben genannten EP-A-686 674 beschrieben.

Aus der DE-A-196 39 229 ist eine Zusammensetzung bekannt, in der mindestens eine Matrix, die Pigmente mit vom Betrachtungswinkel abhängiger Farbigkeit enthält, räumlich oder flächenförmig strukturiert in Form von Strukturelementen in mindestens einer weiteren Matrix vorliegt. Hierbei sind die Matrizes 1 und 2 nicht identisch oder enthalten nichtidentische Pigmente in identischen Konzentrationen. Die Herstellung der Pigmente erfolgt wie in der oben genannten EP-A-686 674 beschrieben.

Die DE-A-196 39 165 beschreibt ein Verfahren zur Erzielung neuer Farbeffekte mittels Pigmenten mit vom Betrachtungswinkel abhängiger Farbigkeit in einer Matrix, wobei die Pigmente nach ihrem Einbringen in die Matrix gezielt raum- und/oder flächenselektiv in der Matrix verkippt werden. Das Verkippen der Pigmente erfolgt vorzugsweise mittels unterschiedlich gerichteter Bewegung der Pigmente in der Matrix oder durch Verwendung von Pigmenten unterschiedlicher Konzentration in der Matrix. Die Herstellung der Pigmente erfolgt wie in der oben genannten EP-A-686 674 beschrieben.

Die DE-A-195 02 413 beschreibt ein Pigment mit vom Betrachtungswinkel abhängiger Farbigkeit, das durch dreidimensionales Vernetzen von orientierten Substanzen flüssigkristalliner Struktur mit chiraler Phase erhalten worden ist. Um ein solches Pigment farbhaltig gegenüber erhöhten Temperaturen zu machen, wird vorgeschlagen, dass das Vernetzen in Gegenwart von zumindest einer weiteren, zumindest zwei vernetzbare Doppelbindungen enthaltenden, farbneutralen Verbindungen durchgeführt wird. Das Auftragen der unvernetzten cholesterischen Mischung erfolgt durch Rakeln, beispielsweise auf eine Folie. Über die Dicke der aufgerakelten Schicht werden keine Angaben gemacht.

Die US-A-5 364 557 beschreibt cholesterische Tinten und Druckfarben, die plättchen- oder schuppenförmige cholesterische Pigmente enthalten. Die Herstellung der Pigmente erfolgt durch Beschichtung eines Transportbandes mit einer cholesterischen Schmelze und nachfolgende Glättung und Ausrichtung des cholesteren Films mittels einer Messerklinge. Die Pigmente können zwei Schichten unterschiedlicher Händigkeit oder zwei Schichten gleicher Händigkeit mit einer dazwischen liegenden weiteren Schicht umfassen, die die Drehrichtung des circular polarisierten Lichts umkehrt. Eine Licht absorbierende Schicht wird nicht als möglicher Bestandteil eines Schichtpigments beschrieben, zumal eine solche Schicht dem gewünschten Effekt einer 100 %-igen Lichtreflexion entgegenwirken würde.

Aus der US-A-5 599 412 ist ein Herstellungsverfahren und eine Apparatur zur Herstellung von cholesterischen Tinten und Druckfarben bekannt. Das geschmolzene Polymer wird, wie in der zuvor zitierten US-A-5 364 557 beschrieben, aufgetragen und ausgerichtet.

Die DE-A-44 18 076 beschreibt Effektlacke bzw. Effektlackierungen, die Interferenzpigmente aus veresterten Celluloseethern enthalten. Mit diesen Interferenzpigmenten sollen sich bei dem Lack von der Lichteinfallsrichtung und von der Betrachtungsrichtung abhängige Farbwechsel oder besonders intensive Farbtöne mit bisher nicht bekannter Brillanz auf dem damit lackierten Gegenstand herstellen lassen. Die Pigmente werden durch Zerkleinern cholesterischer Schichten hergestellt, die nach dem Aushärten eine Dicke von 5 bis 200 µm haben sollen. Die Auftragung der Schichten erfolgt beispielsweise durch Rakeln. In den Beispielen wird eine Schichtdicke von etwa 10 µm angegeben.

Die DE-A-196 297 61 beschreibt kosmetische oder pharmazeutische Zubereitungen, die Pigmente mit vom Betrachtungswinkel abhängiger Farbigkeit enthalten. Die Pigmente enthalten mindestens eine orientierte vernetzte Substanz mit flüssigkristalliner Struktur und mit chiraler Phase. Die Pigmente sind von plättchenförmiger Gestalt und weisen eine Dicke von 1 bis 20 µm auf. Die Herstellung der Pigmente erfolgt, wie in der oben genannten EP-A-686 674 beschrieben, wobei die Schichtdicke jedoch 5 µm statt 7 µm betragen soll.

Aus der WO 96/02597 ist ein Verfahren zur Beschichtung oder zum Bedrucken von Unterlagen mit einem Mittel bekannt, das ein chirales oder achirales, flüssigkristallines Monomer und eine nichtflüssigkristalline, chirale Verbindung enthält. Die flüssigkristallinen Monomere sind vorzugsweise photochemisch polymerisierbare Bisacrylate. Um die für die Ausbildung der erwünschten optischen Eigenschaften notwendige einheitliche Orientierung der cholesterischen Phase auch auf komplex geformten, großen Oberflächen zu erreichen, ist die Beimischung eines polymeren Bindemittels notwendig. Die Herstellung der Schichten, über deren Dicke nichts gesagt wird, erfolgt durch verschiedene Druckverfahren oder durch Spritzen.

Aus der DE-A-196 02 795 ist ein Verfahren zur Herstellung von Pigmentpartikeln bekannt. Die Pigmentpartikel weisen eine weitgehend einheitliche, definierte Form und Größe auf, da sie entweder durch Polymerisation einer polymerisierbaren Mischung in einheitlich dimensionierten Vertiefungen oder durch Vorformung mittels eines Druckverfahrens und nachfolgende Polymerisation hergestellt werden. Die Schichtdicke der Pigmente wird nicht erwähnt.

Die DE-A-196 02 848 beschreibt ein Verfahren zur Herstellung von Pigmenten, wobei eine polymerisierbare Mischung eingesetzt wird, die unter anderem zwingend ein polymeres Bindemittel und/oder monomere Verbindungen, die durch Polymerisation in ein polymeres Bindemittel überführt werden, und/oder ein Dispergierhilfsmittel enthält. Diese Hilfsmittel sollen eine beträchtliche Verbesserung der Fließviskosität bewirken.

Aus der DE-A-42 40 743 sind Pigmente mit vom Betrachtungswinkel abhängiger Farbigkeit bekannt. Die Pigmente werden bevorzugt aus dreidimensional vernetzbaren Polyorganosiloxanen hergestellt, wobei die Flüssigkristallmasse auf eine Metall-, Kunststoff- oder Glasunterlage aufgerakelt, thermisch oder photochemisch vernetzt und das Vernetzungsprodukt dann von der Unterlage abgelöst wird. Die Pigmente weisen vorzugsweise eine Dicke von 5 bis 50 µm auf.

Die EP-B-383 376 beschreibt Flüssigkristallpigmente, die plättchenförmige Trägerpartikel umfassen, die wenigstens teilweise mit flüssigkristallinem Material beschichtet sind. Die Beschichtung erfolgt durch Dispergieren der plättchenförmigen Partikel in einem Lösungsmittel, in dem flüssigkristallines Material gelöst ist und nachfolgendes Ausfällen wenigstens eines Teils des flüssigkristallinen Materials auf die Partikel. Die plättchenförmigen Trägerpartikel werden dabei mit dem Cholester teilweise oder vollständig umhüllt. Gleichmäßige und exakt parallel zur mittleren Schicht angeordnete cholesterische Schichten sind mittels dieses Verfahrens nicht herstellbar. Die Pigmente sind offensichtlich auch nicht volldeckend, da sie vorzugsweise auf schwarze Oberflächen aufgetragen werden sollen.

Die DE-A-196 19 973 skizziert, in nicht nacharbeitbarer Weise, ein Konzept zwei- bzw. dreischichtiger plättchenförmiger Interferenzpigmente. Die Pigmente sollen zumindest eine Lage aufweisen, die aus flüssigkristallinen Polymeren besteht, deren Mesogene zumindest näherungsweise chiral-nematisch und/oder smektisch und/oder cholesterisch geordnet sind. Außerdem ist in den Interferenzpigmenten eine lichtabsorbierende Lage vorgesehen, die zumindest für einen Teil des sichtbaren Lichtspektrums absorbierend ist. Die Pigmente sollen durch Rakeln, Aufwalzen oder Aufsprühen auf eine glatte Unterlage, Aushärten des so erzeugten dünnen Filmes, Aufbringen der lichtabsorbierenden Lage, Aushärten dieser lichtabsorbierenden Lage, gegebenenfalls Aufbringen und Aushärten eines weiteren, nach Zusammensetzung und Lagenstärke mit dem ersten Film übereinstimmenden Filmes, sowie Abziehen und Zerkleinern des ausgehärteten Lagenverbundes erhältlich sein. Konkrete Pigmente werden jedoch nicht offenbart. Im Hinblick auf die stoffliche Zusammensetzung der Pigmente wird lediglich gesagt, dass als flüssigkristalline Polymere "flüssigkristalline Hauptoder Seitenketten-Polymere oder Mischungen davon, flüssigkristalline Oligomere oder Oligomermischungen oder flüssigkristalline Monomere oder Monomermischungen in Betracht" kommen. Beispiele zur Herstellung der Pigmente oder die Pigmente enthaltender Lackformulierungen fehlen. Die Offenbarung der DE-A-196 19 973 erschöpft sich daher in rein theoretischen Erörterungen des Konzeptes zwei- oder dreischichtiger Pigmente. Es

wird somit keine für den Fachmann nacharbeitbare technische Lehre bereitgestellt.

Die WO 94/22976 und die ihr zugrundeliegende GB-A-2276883 beschreiben zweischichtige cholesterische Pigmente auf der Basis von zwei verschiedenen Polyorganosiloxanen der Firma Wacker. Die Herstellung der Pigmente erfolgt in extrem aufwendiger Weise durch separates Beschichten zweier zuvor mit Nylon beschichteter Glasplatten mit Lösungen der oben genannten Flüssigkristalle; Reiben jeder Flüssigkristallschicht, um diese zu orientieren; Anbringen thermisch deformierbarer Spacer auf den Glasplatten; Aufeinanderlegen der Glasplatten mit einander zugewandten cholesterischen Schichten und Vereinigung der cholesterischen Schichten durch thermische Deformation der Spacer bei erhöhter Temperatur im Vakuum sowie Vernetzen der vereinigten Cholesterschichten. Der so erhältliche Film soll, wie auch die aus ihm durch Mahlen erhältlichen Pigmente, eine Dicke von ungefähr 10 µm aufweisen. Der Film löst sich offensichtlich trotz der vorherigen Beschichtung der Glasplatten mit Nylon nur unvollständig von den Glasplatten, so daß Reste des Films zur Gewinnung der Pigmente von den Platten abgekratzt werden müssen, was die Herstellung der Pigmente noch aufwendiger gestaltet. Das Konzept dreischichtiger Pigmente wird lediglich skizziert. Diese Pigmente sind durch das für zweischichtige Pigmente beschriebene Herstellungsverfahren nicht herstellbar. Die WO 94/22976 bzw. die ihr zugrundeliegende GB-A-2276883 stellen somit keine für den Fachmann nacharbeitbare technische Lehre bereit, die Dreischichtpigmente in irgendeiner weise verfügbar machen würde. Die Offenbarung erschöpft sich in rein theoretischen Erörterungen des Aufbaus von dreischichtigen Pigmenten.

Die aus dem Stand der Technik bekannten cholesterischen Interferenzpigmente müssen für die Absorption des transmittierenden Wellenlängenbereiches entweder zusätzliche Pigmente in der Cholestermatrix enthalten oder auf einen farbigen Untergrund aufgebracht werden. Beim Einbau von Fremdpigmenten in die flüssigkristalline Masse ist von Nachteil, dass durch Absorption und Streulicht ein erheblicher Teil des reflektierenden Wellenbereiches absorbiert bzw. gestreut wird, so dass der besondere Vorteil der Interferenzpigmente auf cholesterischer Basis weitgehend aufgehoben wird. Das gleiche Problem tritt auf, wenn man cholesterische Pigmente mit absorbierenden Pigmenten in Lackzubereitungen einmischt. Farbeindruckstörende Reflexionen sind nur vermeidbar, wenn das absorbierende Pigment möglichst feinteilig in die Cholestermatrix eindispergiert wird. Nach allgemeinen Erfahrungen gelingt dies nur, wenn das Pigment mit eigens auf die Pigmentoberfläche abgestimmten Additiven dispergiert wird. Diese Verbindungen, wie z. B. Fettsäuren, Salze der Fettsäuren, Sojalecithine oder Phosphorsäureester stören jedoch die Ausbildung der helikalen Orientierung und verhindern damit eine optimale Farbausbildung. Erfolgt die Absorption dagegen über eine farbige Unterschicht, ist eine gleichmäßig hohe Untergrundqualität notwendig, um den erwünschten Gesamteindruck der Effektbeschichtung zu erzielen. Daher muss ein erheblicher Aufwand zur Vorbehandlung des Untergrundes getrieben werden. Ein idealer Untergrund für maximale Brillanz müsste schwarz/spiegelglänzend beschaffen sein, was beispielsweise bei Autokarosserien nur sehr schwierig zu realisieren ist.

Die ältere EP-A-0 872 336 beschreibt Polymerlaminate bestehend im wesentlichen aus mindestens zwei cholesterischen flüssigkristallinen Polymerschichten und einer dazwischen liegenden lichtabsorbierenden Schicht mit einer Gesamt Schichtdicke von 0,5 bis 100 µm, bevorzugt 1 bis 25 µm, insbesondere 3 bis 15 µm.

Der Erfindung liegt daher die Aufgabe zugrunde, Effektpigmente bereitzustellen, welche die beschriebenen Nachteile des Standes der Technik nicht mehr aufweisen.

Überraschenderweise wurde gefunden, dass die der Erfindung zugrunde liegende Aufgabe durch ein mehrschichtiges Pigment, das mindestens eine transmittierendes Licht teilweise oder vollständig absorbierende Schicht zwischen cholesterischen Schichten eingebaut enthält, gelöst wird.

Gegenstand der vorliegenden Erfindung ist somit ein plättchenförmiges cholesterisches Mehrschichtpigment, das gekennzeichnet ist durch die Schichtfolge $A^1/B/A^2$, wobei

$A^1$ und $A^2$ gleich oder verschieden sind und jeweils mindestens eine cholesterische Schicht umfassen, wobei jede cholesterische Schicht eine Schichtdicke in Bereich von 0,5 µm bis 2 µm aufweist und

B für mindestens eine die Schichten $A^1$ und $A^2$ voneinander trennende Zwischenschicht steht, die das die Schichten $A^1$ und $A^2$ transmittierende Licht teilweise oder vollständig absorbiert, wobei B mindestens ein anorganisches oder organisches Absorptionspigment, gegebenenfalls eingebunden in einer Bindemittelmatrix enthält.

Das erfindungsgemäße Mehrschichtpigment bietet eine Reihe überraschender Vorteile:

a) B kann voll deckend (transmittierendes Licht vollständig absorbierend) eingestellt werden, so dass bei ausreichender Pigmentierungshöhe der Farbeindruck des Pigmentes völlig untergrundunabhängig ist und eine aufwendige und kostspielige auf transparente Interferenzpigmente abgestimmte Untergrundbehandlung, wie bislang üblich, entfällt. Selbstdeckende cholesterische Effektpigmente werden somit erstmals zur Verfügung gestellt.

b) Die Farbe von B kann variiert werden, wodurch eine weitere Steuergröße für den Farbeindruck des Gesamtpigmentes zur Verfügung gestellt wird.

c) Die Brillanz des Gesamtpigments lässt sich durch Variation des Glanzes bzw. der Rauhigkeit von B zusätzlich einstellen.

d) B kann anwendungsspezifisch zur Einstellung der Härte bzw. der Flexibilität des Gesamtpigments variiert werden.

e) B kann elektrisch leitfähig sein und dadurch dem Gesamtpigment elektrische Leitfähigkeit verleihen, ohne dass die Qualität der cholesterischen Schichten dadurch beeinträchtigt wird.

f) $A^1$, B und $A^2$ sind von gleichmäßiger Dicke und parallel zueinander übereinander gestapelt, bilden also eine Art Sandwichstruktur, wodurch die Brillanz des Pigments erheblich verstärkt wird. Außerdem wird dadurch ein verbesserter Farbeindruck im Vergleich zu rundum beschichteten Pigmenten bewirkt, weil sämtliche Cholesteren-Moleküle einer Schicht gleich ausgerichtet sind.

g) Der Farbeindruck des Pigments ist von externen Stimuli weitgehend unabhängig, d. h. über einen weiten Temperatur- und Druckbereich stabil.

Die obere und die untere cholesterische Schicht $A^1$ bzw. $A^2$ des erfindungsgemäßen Pigments besitzen gleiche oder verschiedene optische Eigenschaften. Sie können insbesondere Licht gleicher oder unterschiedlicher Wellenlänge reflektieren, d. h. von gleicher oder von unterschiedlicher Farbe sein. Im letztgenannten Fall lassen sich besonders interessante Farbeffekte erzielen. Besonders bevorzugt sind $A^1$ und $A^2$ von unterschiedlicher Händigkeit, so dass beispielsweise $A^1$ Licht einer bestimmten Wellenlänge linkszirkular-polarisiert reflektiert, wogegen $A^2$ Licht derselben Wellenlänge rechtszirkular-polarisiert reflektiert. Vorteilhafterweise erscheint deshalb beispielsweise ein Lack, der erfindungsgemäße Pigmente in dieser bevorzugten Ausführungsform enthält, besonders brillant, da in der Lackschicht $A^1$ und $A^2$ in statistischer Verteilung dem einfallenden Licht zugewandt sind, so dass der Lack sowohl rechts- als auch linkszirkular-polarisiertes Licht einer bestimmten Wellenlänge reflektiert, wogegen ein Lack, der nur Pigmente mit nur einer cholesterischen Schicht oder mit mehreren cholesterischen Schichten gleicher Händigkeit enthält, entweder das links- oder das rechtszirkular-polarisierte Licht durchlässt.

$A^1$ und $A^2$ können auch hinsichtlich ihrer mechanischen Eigenschaften gleich oder verschieden sein. Sie können beispielsweise unterschiedliche Dicke oder Sprödigkeit aufweisen.

B enthält mindestens ein anorganisches oder organisches Absorptionspigment, vorzugsweise eingebunden in einer Bindemittelmatrix. Das Absorptionspigment kann ein weiß-, Bunt- oder vorzugsweise ein Schwarzpigment sein. Geeignete anorganische Absorptionspigmente sind beispielsweise Titandioxid, $Al_2O_3$, Bariumsulfat, Strontiumsulfat, Zinkoxid, Zinkphosphate, schwarzes Eisenoxid, Blei-chromat, Strontiumchromat, Bariumchromat sowie metallische Pigmente wie Aluminium- oder Bronzepulver.

Geeignete organische Absorptionspigmente sind beispielsweise Azopigmente, Metallkomplex-Pigmente, wie Azo- und Azomethin-Metallkomplexe, Isoindolinon- und Isoindolin-Pigmente, Phthalocyanin-Pigmente, Chinakridon-Pigmente, Perinon- und Perylen-Pigmente, Anthrachinon-Pigmente, Diketopyrrolopyrrol-Pigmente, Thioindigo-Pigmente, Dioxazin-Pigmente, Triphenylmethan-Pigmente, Chinophthalon-Pigmente und fluoreszierende Pigmente.

Besonders geeignet sind feinteilige Absorptionspigmente mit einer mittleren Teilchengröße von 0,01 bis 1 µm, vorzugsweise 0,01 bis 0,1 µm.

Bevorzugt einsetzbar sind Graphitpigmente oder verschiedene Rußtypen, ganz besonders bevorzugt leicht dispergierbare Farbruße mit einer spezifischen Oberfläche von 30 bis 150 $m^2/g$ (BET-Methode) und einem Absorptionsvermögen von 50 bis 100 ml Dibutylphthalat/100 g (DBP-Zahl).

Besonders bevorzugte Absorptionspigmente sind solche, die der transmittierendes Licht absorbierenden Schicht B magnetische Eigenschaften verleihen. Hierfür geeignet sind beispielsweise $\gamma$-$Fe_2O_3$, $Fe_3O_4$, $CrO_2$ oder ferromagnetische Metallpigmente, wie z. B. Fe-, Fe-Cu-, Fe-Ni-Co-Legierungen. Mit diesen Pigmenten lassen sich hochglänzende schwarze Zwischenschichten herstellen.

Pigmente, deren absorbierende Schicht magnetisch ist, können vorteilhafterweise durch Anlegen eines Magnetfeldes beliebig orientiert werden. Beispielsweise kann so vermieden werden, dass einzelne Pigmentplättchen aus den anderen herausragen, was zur Folge hat, dass weniger Licht gestreut wird und der Farbeindruck besser wird. Alle Plättchen können gemeinsam in einem bestimmten Winkel orientiert werden. Es ist auch möglich, gesamtflächige Raster zum Erzielen neuer Farbeffekte oder partielle Raster zum optischen Herausheben von Schriften oder Strukturen zu erzeugen. Die erfindungsgemäßen, magnetischen cholesterischen Pigmente sind auch in einer flüssigen Matrix vorteilhaft einsetzbar, beispielsweise in LCDs, in denen sie bei Anlegen eines Magnetfeldes ihre Richtung und somit ihren Farbeindruck ändern.

Die Absorptionspigmente sind vorzugsweise in eine organische Bindemittelmatrix eingebunden. Als Bindemittel können die lacküblichen Systeme eingesetzt werden. Vorzugsweise eignen sich strahlungshärtbare Systeme, die reaktive, vernetzungsfähige Gruppen, wie Acryl-, Methacryl-, α-Chloracryl-, Vinyl-, Vinylether-, Epoxid-, Cyanat-, Isocyanat- oder Isothiocyanatgruppen enthalten.

Als Bindemittel sind auch monomere Mittel und deren Mischungen mit polymeren Bindemitteln einsetzbar. Als monomere Mittel kommen bevorzugt solche in Betracht, die zwei oder mehr vernetzungsfähige Gruppen wie Acryl-, Methacryl-, α-Chloracryl-, Vinyl-, Vinylether-, Epoxid-, Cyanat-, Isocyanat- oder Isothiocyanatgruppen enthalten. Besonders bevorzugt sind Acryl-, Methacryl- oder Vinylethergruppen. Monomere Mittel mit zwei vernetzungsfähigen Gruppen sind beispielsweise die Diacrylate, die Divinylether oder die Dimethacrylate von Diolen wie beispielsweise Propandiol, Butandiol, Hexandiol, Ethylenglykol, Diethylenglykol, Triethylenglykol oder Tetrapropylenglykol.

Monomere Mittel mit drei vernetzungsfähigen Gruppen sind beispielsweise die Triacrylate, die Trivinylether oder die Trimethacrylate von Triolen wie beispielsweise Trimethylolpropan, ethoxyliertem Trimethylolpropan mit 1 bis 20 Ethylenoxideinheiten, propoxyliertem Trimethylolpropan mit 1 bis 20 Propylenoxideinheiten, gemischt ethoxyliertem und propoxyliertem Trimethylolpropan bei dem die Summe aus Ethylenoxideinheiten und Propylenoxideinheiten 1 bis 20 beträgt. Monomere Mittel mit drei vernetzungsfähigen Gruppen sind beispielsweise auch die Triacrylate, die Trivinylether oder die Trimethacrylate von Glycerin, ethoxyliertem Glycerin mit 1 bis 20 Ethylenoxideinheiten, propoxyliertem Glycerin mit 1 bis 20 Propylenoxideinheiten, gemischt ethoxyliertem und propoxyliertem Glycerin, bei dem die Summe aus Ethylenoxideinheiten und Propylenoxideinheiten 1 bis 20 beträgt.

Monomere Mittel mit vier vernetzungsfähigen Gruppen sind beispielsweise die Tetraacrylate, die Tetravinylether oder die Tetramethacrylate von Tetraolen wie beispielsweise Bis-trimethylolpropan, ethoxyliertem Bis-trimethylolpropan mit 1 bis 20 Ethylenoxideinheiten, propoxyliertem Bis-trimethylolpropan mit 1 bis 20 Propylenoxideinheiten, gemischt ethoxyliertem und propoxyliertem Bis-trimethylolpropan, bei dem die Summe aus Ethylenoxideinheiten und Propylenoxideinheiten 1 bis 20 beträgt. Monomere Mittel mit vier vernetzungsfähigen Gruppen sind beispielsweise auch die Tetraacrylate, die Tetravinylether oder die Tetramethacrylate von Tetraolen wie beispielsweise Pentaerythrit, ethoxyliertem Pentaerythrit mit 1 bis 20 Ethylenoxideinheiten, propoxyliertem Pentaerythrit mit 1 bis 20 Propylenoxideinheiten, gemischt ethoxyliertem und propoxyliertem Pentaerythrit, bei dem die Summe aus Ethylenoxideinheiten und Propylenoxideinheiten 1 bis 20 beträgt.

Zur Erhöhung der Reaktivität bei der Vernetzung oder Polymerisation an Luft können die Bindemittel und die monomeren Mittel 0,1 bis 10 % eines primären oder sekundären Amins enthalten. Beispiele für geeignete Amine sind Ethanolamin, Diethanolamin oder Dibutylamin.

Besonders bevorzugt sind die Absorptionspigmente der Schicht B in eine Bindemittelmatrix eingebunden, die die weiter unten als Inhaltsstoffe der Schichten $A^1$ und $A^2$ beschriebenen cholesterischen Gemische enthält. Ganz besonders bevorzugt umfasst die Bindemittelmatrix der Schicht B die gleichen cholesterischen Gemische wie die Schichten $A^1$ und $A^2$.

Die Herstellung der Absorptionspigmentformulierung kann nach den üblichen, in der Fachwelt bekannten Dispergierverfahren und unter Verwendung von Verdünnungsmitteln, Dispergiermitteln, Photoinitiatoren und gegebenenfalls weiteren Additiven erfolgen.

Als Verdünnungsmittel können Wasser oder organische Flüssigkeiten oder deren Mischungen verwendet werden, wobei organische Flüssigkeiten bevorzugt sind. Besonders bevorzugt sind organische Flüssigkeiten mit einem Siedepunkt unterhalb von 140 °C, insbesondere Ether wie Tetrahydrofuran, Ketone wie Ethylmethylketon und Ester wie Butylacetat.

Als Dispergiermittel können niedermolekulare Dispergiermittel wie beispielsweise Stearinsäure oder auch polymere Dispergiermittel verwendet werden. Geeignete polymere Dispergiermittel oder Dispergierharze sind dem Fachmann bekannt. Insbesondere sind zu nennen sulfonatgruppen-, phosphatgruppen-, phosphonatgruppen- oder carboxylgruppenhaltige Polyurethane, carboxylgruppenhaltige Vinylchloridcopolymere, Polyiminpolyester oder Polyetheracrylate mit oder ohne eingebaute funktionelle Gruppen.

Für die Herstellung von vernetzbaren oder polymerisierbaren Absorptionspigmentformulierungen können für die photochemische Polymerisation handelsübliche Photoinitiatoren verwendet werden, beispielsweise die weiter unten für die photochemische Polymerisation der cholesterischen Gemische aufgeführten Photoinitiatoren.

Die Schichtdicke jeder einzelnen Schicht von B beträgt etwa 0,2 bis 5 µm, insbesondere etwa 0,5 bis 3 µm. Der Durchmesser der erfindungsgemäßen Pigmente beträgt etwa 5 bis 500 µm, insbesondere etwa 10 bis 100 µm, besonders bevorzugt etwa 10 bis 30 µm. In der Regel beträgt der Pigmentdurchmesser etwa das 5-fache der Pigmentdicke.

Vorzugsweise enthalten $A^1$ und $A^2$ der erfindungsgernäßen Pigmente cholesterische Gemische, die ausgewählt sind unter

a) mindestens einem cholesterischen, polymerisierbaren Monomer;

b) mindestens einem achiralen, nematischen, polymerisierbaren Monomer und einer chiralen Verbindung;

c) mindestens einem cholesterischen, vernetzbaren Oligomer oder Polymer; oder

d) einem cholesterischen Polymer in einem polymerisierbaren Verdünnungsmittel,

e) mindestens einem cholesterischen Polymer, dessen cholesterische Phase durch schnelles Abkühlen unter die Glasübergangstemperatur eingefroren werden kann,

in gehärtetem Zustand.

Durch Härtung wird die gleichmäßige Orientierung der cholesterischen Moleküle in der Cholesterschicht fixiert.

Bevorzugte Monomere der Gruppe a) sind in der DE-A-196 02 848 beschrieben, auf die hiermit in vollem Umfang Bezug genommen wird. Insbesondere umfassen die Monomere a) mindestens ein chirales, flüssigkristallines, polymerisierbares Monomer der Formel I

$$\left[ Z^1 - Y^1 - A^1 - Y^2 - M^1 - Y^3 - \right]_n X \qquad (I)$$

in der die Variablen die folgende Bedeutung haben:

| | |
|---|---|
| $Z^1$ | eine polymerisierbare Gruppe oder ein Rest, der eine polymerisierbare Gruppe trägt, |
| $Y^1, Y^2, Y^3$ | unabhängig voneinander chemische Bindungen, Sauerstoff, Schwefel, —CO—O—, —O—CO—, —O—CO—O— , —CO—N(R)— oder —N(R)—CO— , |
| $A^1$ | ein Spacer, |
| $M^1$ | eine mesogene Gruppe, |
| x | ein n-wertiger chiraler Rest, |
| R | Wasserstoff oder $C_1$-$C_4$-Alkyl, |
| n | 1 bis 6 |

wobei die Reste $Z^1$, $Y^1$, $Y^2$, $Y^3$, $A^1$ und $M^1$, gleich oder verschieden sein können, wenn n größer als 1 ist.

Bevorzugte Reste $Z^1$ sind:
$CH_2 = CH-$ , $CH \equiv C-$ ,

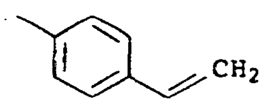 , 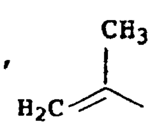 , 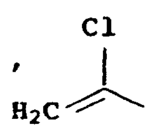 ,

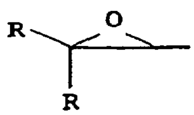 , 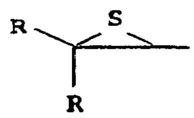 , 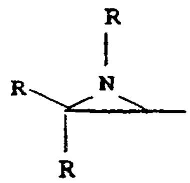 , 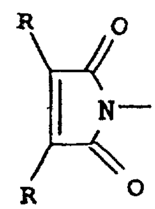

—N=C=O, —N=C=S, —O—C≡N,
—COOH, —OH oder —$NH_2$,
wobei die Reste R gleich oder verschieden sein können und Wasserstoff oder $C_1$-$C_4$-Alkyl, wie Methyl, Ethyl, n-Propyl, iso-Propyl, n-Butyl, iso-Butyl, sec.-Butyl oder tert.-Butyl, bedeuten. Von den reaktiven polymerisierbaren Gruppen können die Cyanate spontan zu Cyanuraten trimerisieren und sind daher bevorzugt. Die anderen genannten Gruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Carboxylgruppen können zu Polyestern und Polyamiden kondensiert werden. Die Maleinimidogruppe eignet sich besonders zur radikalischen Copolymerisation mit olefinischen Verbindungen wie Styrol. Die komplementären reaktiven Gruppen können dabei entweder in einer zweiten erfindungsgemäßen Verbindung vorhanden sein, die mit der ersteren gemischt wird, oder sie können durch Hilfsverbindungen, die 2 oder mehr dieser komplementären Gruppen enthalten, in das polymere Netzwerk eingebaut werden.

Besonders bevorzugte Gruppierungen $Z^1$-$Y^1$ sind Acrylat und Methacrylat.

$Y^1$-$Y^3$ können die oben genannten Bedeutungen haben, wobei unter einer chemischen Bindung eine kovalente Einfachbindung verstanden werden soll.

Als Spacer $A^1$ kommen alle für diesen Zweck bekannten Gruppen in Betracht. Die Spacer enthalten in der Regel 1 oder mehrere, wie z. B. 2 bis 30, vorzugsweise 1 bis 12 oder 2 bis 12 C-Atome und bestehen aus linearen aliphatischen Gruppen. Sie können in der Kette z.B. durch O, S, NH oder $NCH_3$ unterbrochen sein, wobei diese Gruppen nicht benachbart sein dürfen. Als Substituenten für die Spacerkette kommen dabei noch Fluor, Chlor, Brom, Cyan, Methyl oder Ethyl in Betracht.

Repräsentative Spacer sind beispielsweise:
$-(CH_2)_p-$, $-(CH_2CH_2O)_mCH_2CH_2-$, $-CH_2CH_2SCH_2CH_2-$, $-CH_2CH_2NHCH_2CH_2-$,

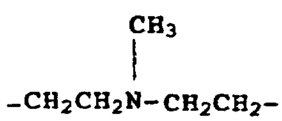 , 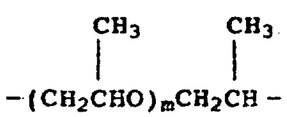 , 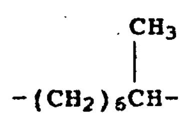 oder 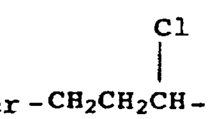 ,

wobei

m für 1 bis 3 und
p für 1 bis 12 steht.

Die mesogene Gruppe $M^1$ hat vorzugsweise die Struktur

$$(T\text{-}Y^8)_s\text{-}T$$

wobei $Y^8$ ein Brückenglied gemäß einer der Definitionen von $Y^1$, s eine Zahl von 1 bis 3 und T gleiche oder verschiedene zweiwertige isocycloaliphatische, heterocycloaliphatische, isoaromatische oder heteroaromatische Reste bedeuten.

Die Reste T können auch durch $C_1$-$C_4$-Alkyl, Fluor, Chlor, Brom, Cyan, Hydroxy oder Nitro substituierte Ringsysteme sein. Bevorzugte Reste T sind:

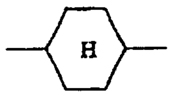 , 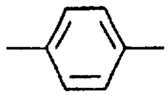 , 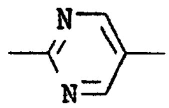 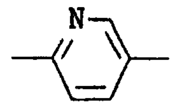 ,

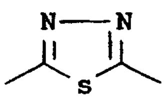 , 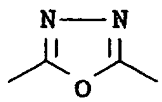 , 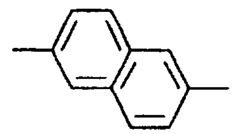 ,

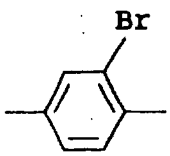 , 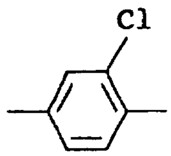 , 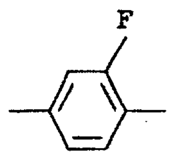 , 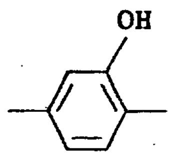 , 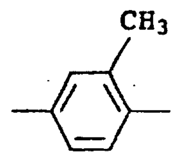 ,

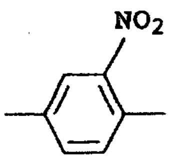 , 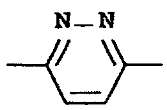 und 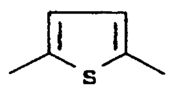 .

Besonders bevorzugt sind die folgenden mesogenen Gruppen $M^1$:

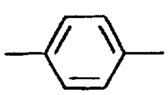 , 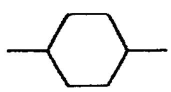 , 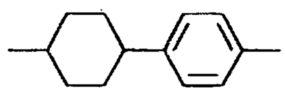 , 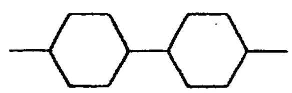 ,

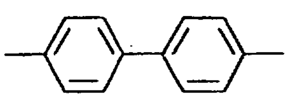 , 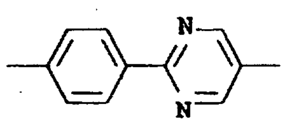 , 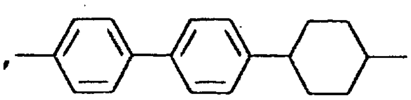 ,

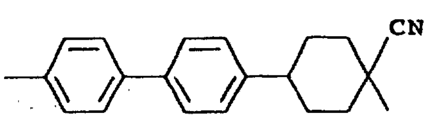 , 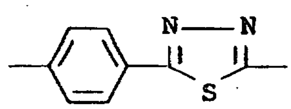 ,

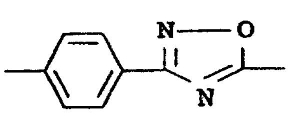 , 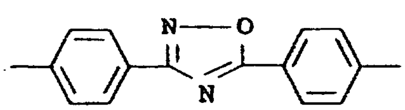 ,

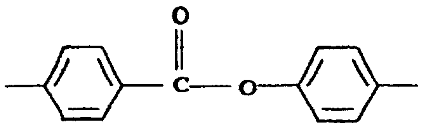 , 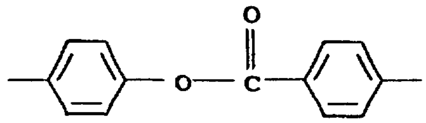 ,

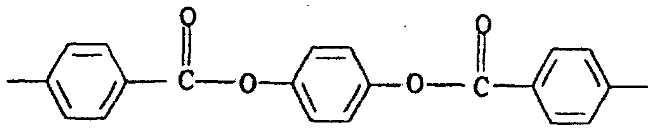 ,

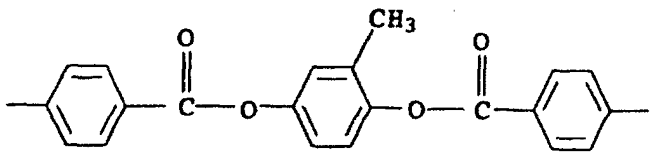 ,

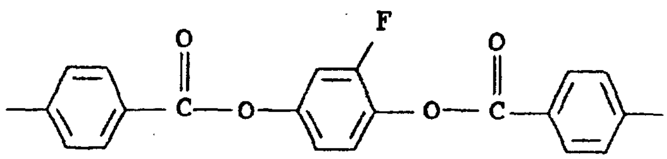 ,

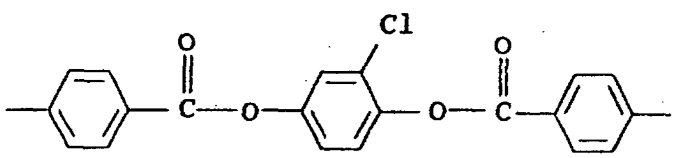

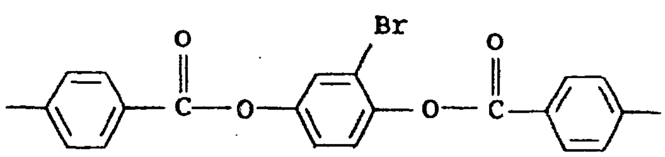 ,

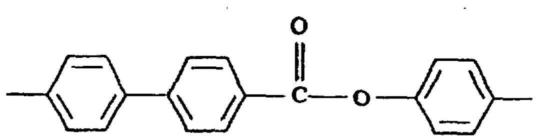 ,

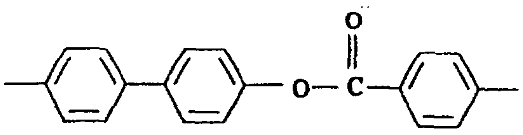 , 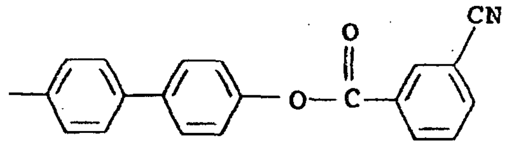 ,

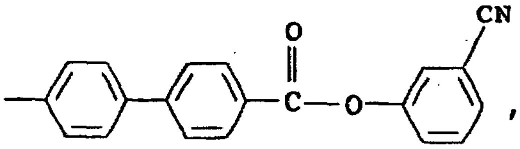 , 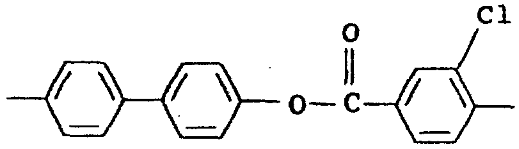 ,

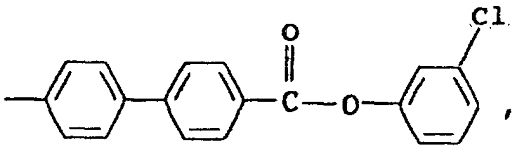 , 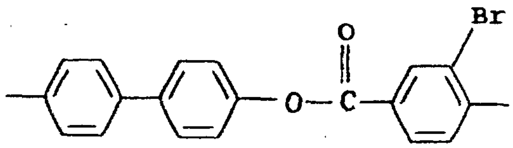 ,

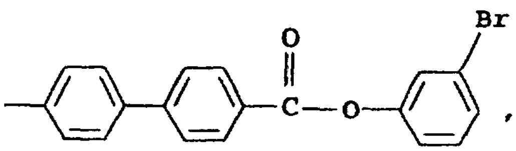 , 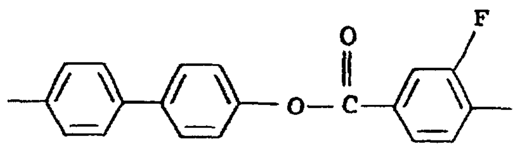 .

Von den chiralen Resten X der Verbindungen der Formel I sind u.a. aufgrund der Verfügbarkeit insbesondere solche bevorzugt, die sich von Zuckern, Binaphthyl- oder Biphenylderivaten sowie optisch aktiven Glykolen, Dialkoholen oder Aminosäuren ableiten.

Bei den Zuckern sind insbesondere Pentosen und Hexosen und davon abgeleitete Derivate zu nennen.

Beispiele für Reste X sind die folgenden Strukturen, wobei die endständigen Striche jeweils die freien Valenzen bedeuten.

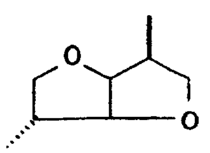

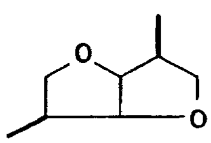

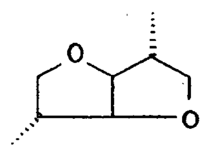

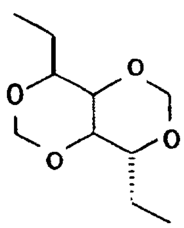

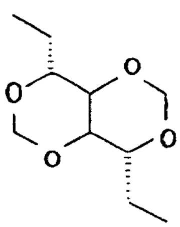

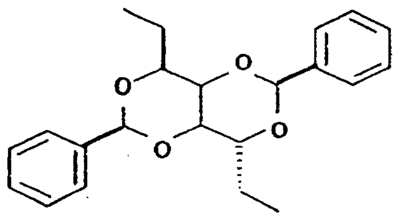

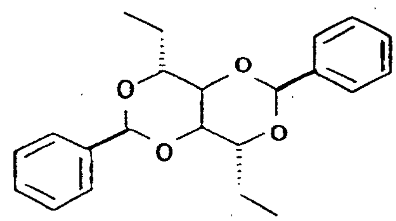

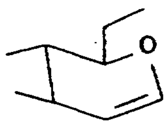

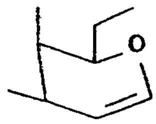

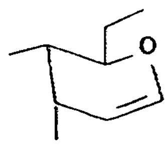

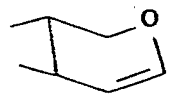

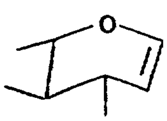

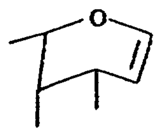

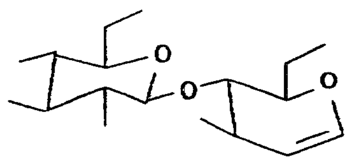

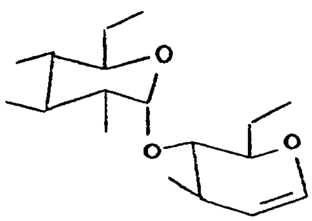

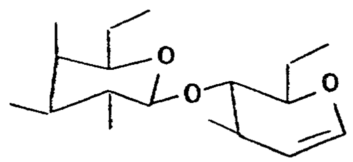

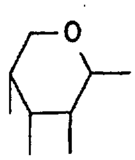

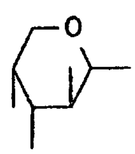

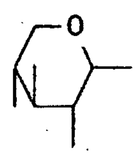

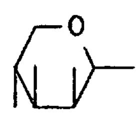

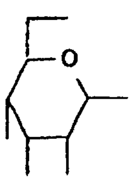

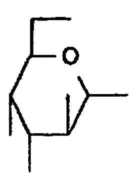

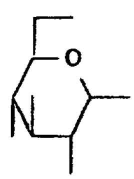

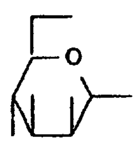

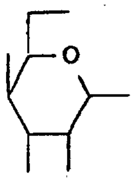 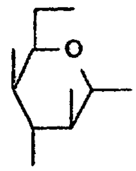 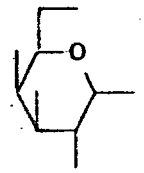 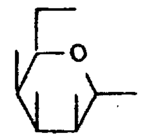

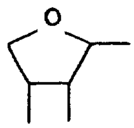 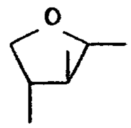 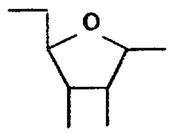 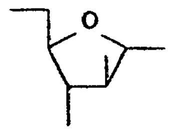 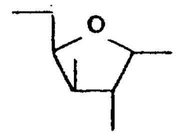

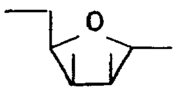 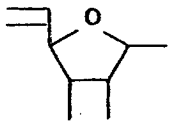 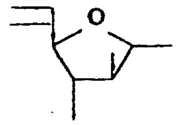 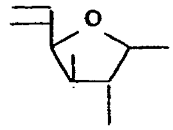 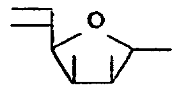

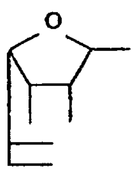 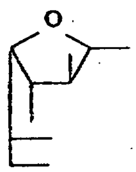 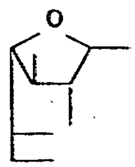 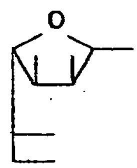

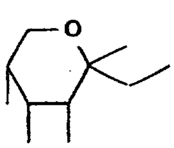 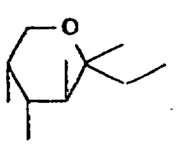 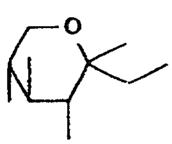 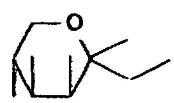 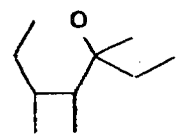

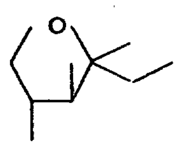 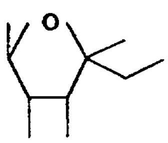 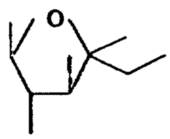 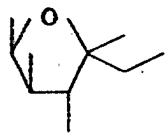 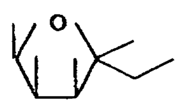

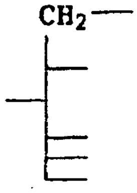 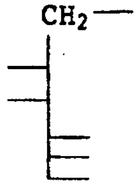 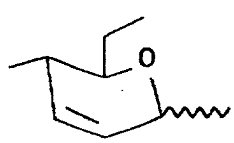 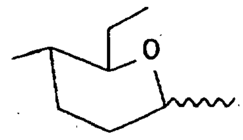

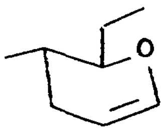 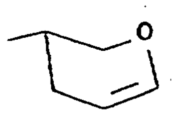 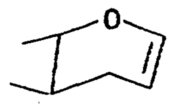 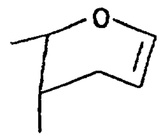

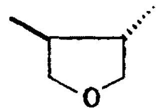

Besonders bevorzugt sind

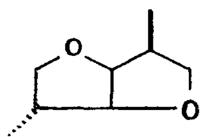 , 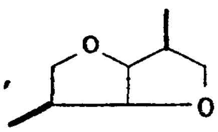 , 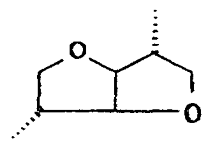 ,

und

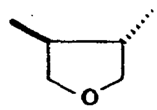 ,

Weiterhin sind auch chirale Gruppen geeignet, die folgende Strukturen aufweisen:

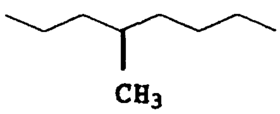 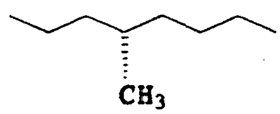 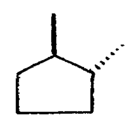

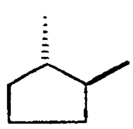 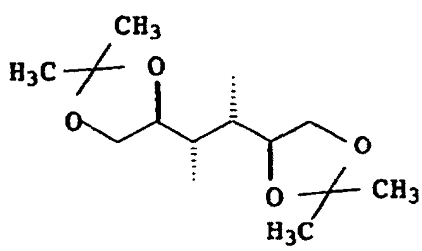

Weitere Beispiele sind in der deutschen Anmeldung P 43 42 280.2 aufgeführt.

n ist bevorzugt 2.

Als bevorzugte Monomere der Gruppe b) enthält das cholesterische Gemisch im erfindungsgemäßen Verfahren mindestens ein achirales flüssigkristallines polymerisierbares Monomer der Formel II

$$Z^2—Y^4—A^2—Y^5—M^2—Y^6—A^3(Y^7—Z^3)_n \quad (II)$$

in der die Variablen die folgende Bedeutung haben:

| | |
|---|---|
| $Z^2$,$Z^3$ | gleiche oder verschiedene polymerisierbare Gruppen oder Reste, die eine polymerisierbare Gruppe enthalten |
| n | 0 oder 1 |
| $Y^4$,$Y^5$,$Y^6$,$Y^7$ | unabhängig voneinander chemische Bindungen, Sauerstoff, Schwefel, —CO—O—,—O—CO—,—O—CO—O— , —CO—N(R)— oder —N(R)—CO— , |
| $A^2$,$A^3$ | gleiche oder verschiedene Spacer und |
| $M^2$ | eine mesogene Gruppe. |

Dabei gelten für die polymerisierbaren Gruppen, die Brückenglieder $Y^4$ bis $Y^7$, die Spacer und die mesogene Gruppe die gleichen Bevorzugungen wie für die entsprechenden Variablen der Formel I.

Außerdem enthält das Gemisch der Gruppe b) eine chirale Verbindung. Die chirale Verbindung bewirkt die Verdrillung der achiralen flüssigkristallinen Phase zu einer cholesterischen Phase. Dabei hängt das Ausmaß der Verdrillung von der Verdrillungsfähigkeit des chiralen Dotierstoffs und von seiner Konzentration ab. Damit hängt also die Ganghöhe der Helix und wiederum auch die Interferenzfarbe von der Konzentration des chiralen Dotierstoffs ab. Es kann daher für den Dotierstoff kein allgemeingültiger Konzentrationsbereich angegeben werden. Der Dotierstoff wird in der Menge zugegeben, bei der der gewünschte Farbeffekt entsteht.

Bevorzugte chirale Verbindungen sind solche der Formel Ia

$$\left[Z^1—Y^1—A^1—Y^2—M^a—Y^3—\right]_n X \qquad \text{(Ia)},$$

in der $Z^1$, $Y^1$, $Y^2$, $Y^3$, $A^1$, X und n die obengenannten Bedeutung haben und $M^a$ ein zweiwertiger Rest ist, der mindestens ein heterooder isocyclisches Ringsystem enthält.

Der Molekülteil $M^a$ ähnelt dabei den beschriebenen mesogenen Gruppen, da auf diese Weise eine besonders gute Kompatibilität mit der flüssigkristallinen Verbindung erreicht wird. $M^a$ muß jedoch nicht mesogen sein, da die Verbindung Ia lediglich durch ihre chirale Struktur eine entsprechende Verdrillung der flüssigkristallinen Phase bewirken soll. Bevorzugte Ringsysteme, die in $M^a$ enthalten sind, sind die oben erwähnten Strukturen T, bevorzugte Strukturen $M^a$ solche der oben genannten Formel $(T\text{-}Y^8)_s\text{-}T$. weitere Monomere und chirale Verbindungen der Gruppe b) sind in der WO 97/00600 und der ihr zugrundeliegenden DE-A-195 324 08 beschrieben, auf die hiermit in vollem Umfang Bezug genommen wird.

Bevorzugte Polymere der Gruppe c) sind cholesterische Cellulosederivate, wie sie in der DE-A-197 136 38 beschrieben werden, insbesondere cholesterische Mischester von

(VI) Hydroxyälkylethern der Cellulose mit
(VII) gesättigten, aliphatischen oder aromatischen Carbonsäuren und
(VIII) ungesättigten Mono- oder Dicarbonsäuren.

Besonders bevorzugt sind Mischester, bei denen die über Etherfunktionen gebundenen Hydroxyalkylreste der Komponente VI geradkettige oder verzweigte $C_2$-$C_{10}$-Hydroxyalkylreste, insbesondere Hydroxypropyl- und/oder Hydroxyethylreste umfassen. Die Komponente VI der erfindungsgemäßen Mischester weist vorzugsweise ein Molekulargewicht von etwa 500 bis etwa 1 Million auf. Bevorzugtermaßen sind die Anhydroglukose-Einheiten der Cellulose mit einem durchschnittlichen molaren Substitutionsgrad von 2 bis 4 mit Hydroxyalkylresten verethert. Die Hydroxyalkylgruppen in der Cellulose können gleich oder verschieden sein. Bis zu 50 % davon können auch durch Alkylgruppen (insbesondere $C_1$-$C_{10}$-Alkylgruppen) ersetzt werden. Ein Beispiel hierfür ist die Hydroxypropylmethylcellulose.

Als Komponente VII der einsetzbaren Mischester sind geradkettige aliphatische $C_1$-$C_{10}$-Carbonsäuren, insbesondere $C_2$-$C_6$-Carbonsäuren, verzweigte aliphatische $C_4$-$C_{10}$-Carbonsäuren, insbesondere $C_4$-$C_6$-Carbonsäuren oder geradkettige oder verzweigte Halogencarbonsäuren brauchbar. Die Komponente VII kann auch Benzoesäure oder aromatisch substituierte aliphatische Carbonsäuren, insbesondere Phenylessigsäure sein. Besonders bevorzugt ist die Komponente VII ausgewählt unter Essigsäure, Propionsäure, n-Buttersäure, Isobuttersäure oder n-Valeriansäure, insbesondere unter Propionsäure, 3-Cl-Propionsäure, n-Buttersäure oder Isobuttersäure.

Vorzugsweise ist die Komponente VIII ausgewählt unter ungesättigten $C_3$-$C_{12}$-Mono- oder Dicarbonsäuren oder Halbestern einer derartigen Dicarbonsäure, insbesondere $\alpha,\beta$-ethylenisch ungesättigten $C_3$-$C_6$-Mono- oder Dicarbonsäuren oder Halbestern der Dicarbonsäuren.

Die Komponente VIII der einsetzbaren Mischester ist besonders bevorzugt ausgewählt unter Acrylsäure, Methacrylsäure, Crotonsäure, Vinylessigsäure, Maleinsäure, Fumarsäure oder Undecensäure, insbesondere unter Acrylsäure oder Methacrylsäure.

Vorzugsweise ist die Komponente VI mit einem durchschnittlichen molaren Substitutionsgrad von 1,5 bis 3, insbesondere von 1,6 bis 2,7, besonders bevorzugt von 2,3 bis 2,6 mit der Komponente VII und VIII verestert. Vorzugsweise sind etwa 1 bis 30 %, insbesondere 1 bis 20 % oder 1 bis 10 %, besonders bevorzugt etwa 5 bis 7 % der OH-Gruppen der Komponente VI mit der Komponente VIII verestert.

Das Mengenverhältnis der Komponente VII zu Komponente VIII bestimmt den Farbton des Polymers.

Gut geeignete Polymere der Gruppe c) sind außerdem die in der DE-A-197 17 371 beschriebenen, propargylterminierten, cholesterischen Polyester oder Polycarbonate.

Weiterhin sind vernetzbare Oligo- oder Polyorganosiloxane einsetzbar, wie sie z. B. aus der EP-A-0 358 208, der DE-A-195 41 820 oder der DE-A- 196 19 460 bekannt sind.

Bevorzugt unter diesen Verbindungen sind Polyester oder Polycarbonate mit wenigstens einer Propargylendgruppe der Formel $R^3C{\equiv}C\text{-}CH_2\text{-}$, worin $R^3$ für H, $C_1$-$C_4$-Alkyl, Aryl oder Ar-$C_1$-$C_4$-alkyl (z. B. Benzyl oder Phenethyl) steht, die direkt oder über ein Bindeglied an die Polyester oder Polycarbonate gebunden ist. Das Bindeglied ist vorzugsweise ausgewählt unter

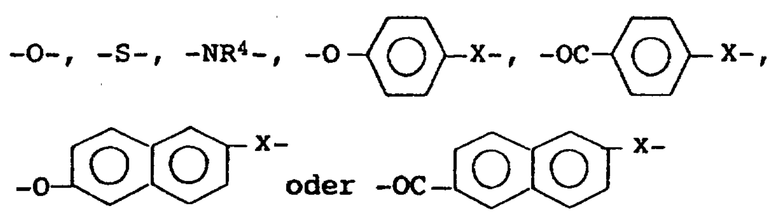
oder

(die Propargylgruppe ist an X gebunden),

worin $R^4$ für H, $C_1$-$C_4$-Alkyl oder Phenyl steht, X für O, S oder $NR^2$ steht, und $R^2$ für H, $C_1$-$C_4$-Alkyl oder Phenyl steht.

Vorzugsweise ist in den Polyestern die Propargylendgruppe über

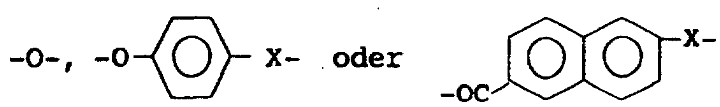
oder (X = O, S)

gebunden.

Die Polyester enthalten vorzugsweise

(IX)mindestens eine aromatische oder araliphatische Dicarbonsäureeinheit und/oder mindestens eine aromatische oder araliphatische Hydroxycarbonsäureeinheit und

(X) mindestens eine Dioleinheit.

Bevorzugte Dicarbonsäureeinheiten sind solche der Formel

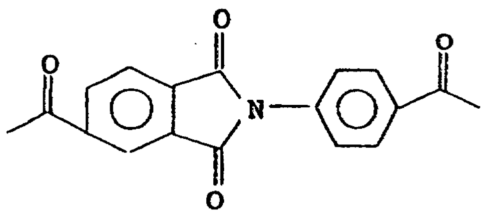 , 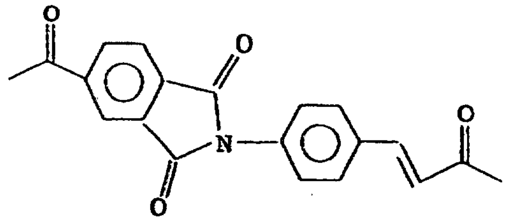 ,

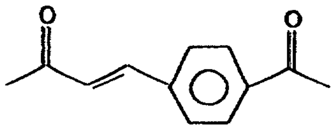 ,

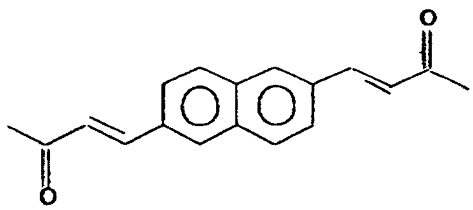 ,

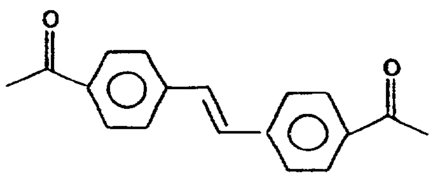 , 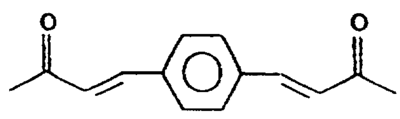 ,

insbesondere solche der Formel

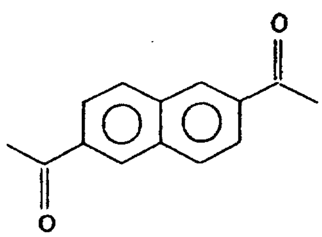 , 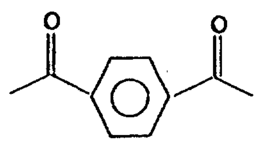 ,

oder 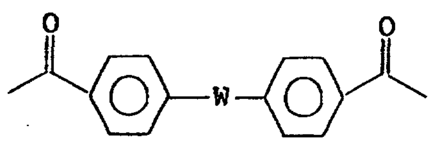 ,

wobei jede der Phenylgruppen bzw. die Naphthylgruppe 1, 2 oder 3 Substituenten aufweisen kann, die unabhängig voneinander ausgewählt sind unter $C_1$-$C_4$-Alkyl, $C_1$-$C_4$-Alkoxy, Halogen oder Phenyl, wobei in den obigen Formeln

| | |
|---|---|
| W | für NR, S, O, $(CH_2)_pO(CH_2)_q$, $(CH_2)_m$ oder eine Einfachbindung steht, |
| R | Alkyl oder Wasserstoff bedeutet, |
| m | eine ganze Zahl von 1 bis 15 bedeutet, und |
| p und q | unabhängig voneinander für ganze Zahlen von 0 bis 10 stehen. |

Bevorzugte Hydroxycarbonsäureeinheiten sind solche der Formel

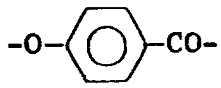 , 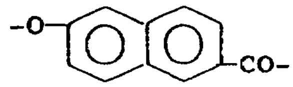

und

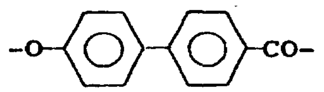 ,

wobei jede Phenylgruppe oder die Naphthylgruppe 1, 2 oder 3 Substituenten aufweisen kann, die unabhängig voneinander ausgewählt sind unter $C_1$-$C_4$-Alkyl, $C_1$-$C_4$-Alkoxy, Halogen oder Phenyl.

Bevorzugte Dioleinheiten sind solche der Formel

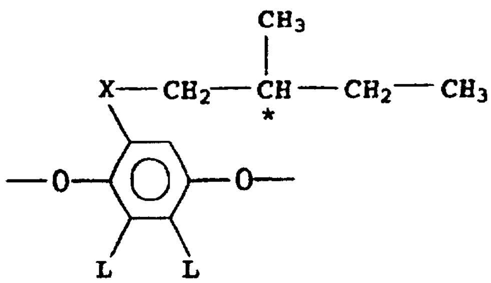 , 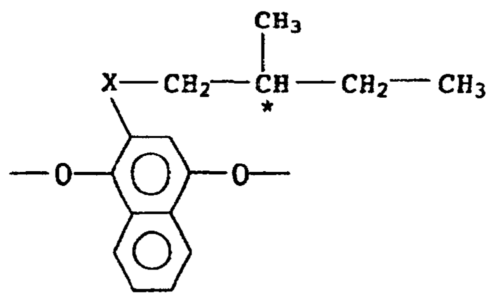 ,

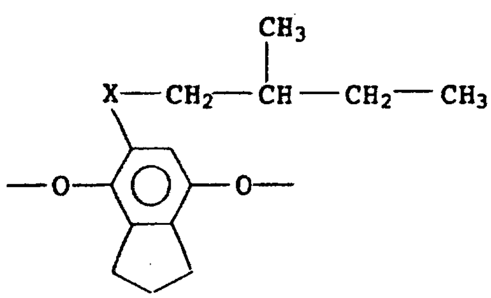 ,

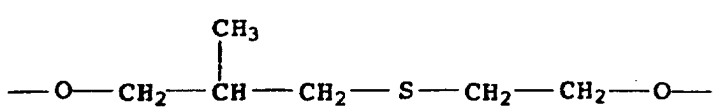 ,

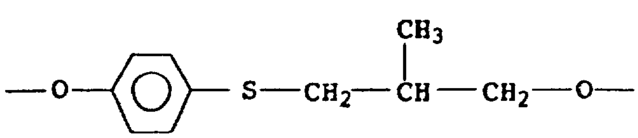 ,

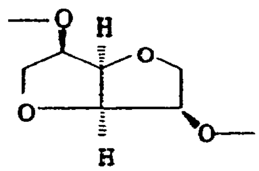 , 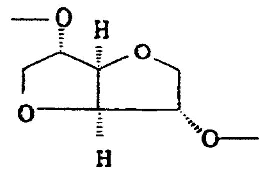 ,

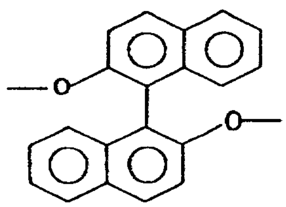 , 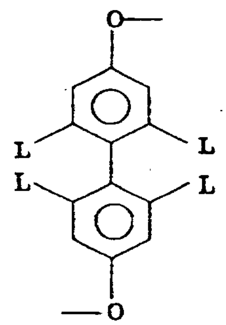 ,

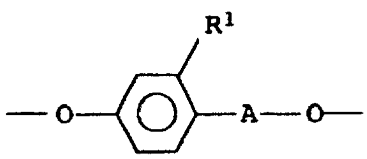 , 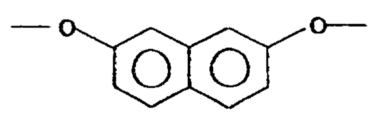 ,

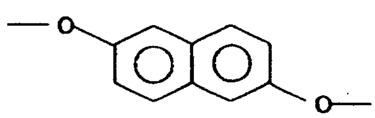 , $—O—(CH_2)_n—O—$ ,

insbesondere solche der Formel

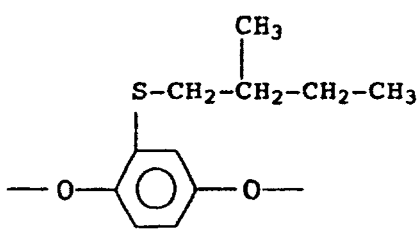 , 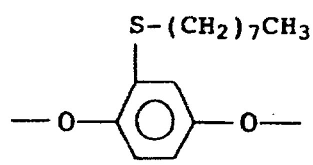 ,

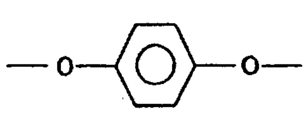 , 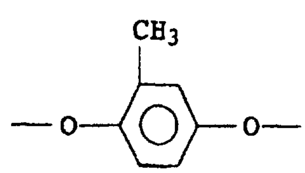 , 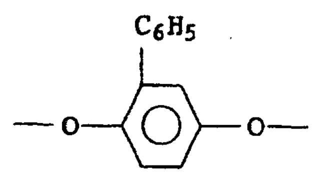 ,

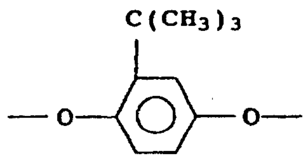, 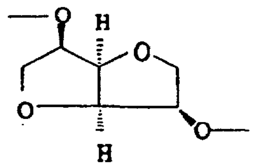 oder 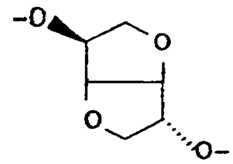,

wobei in den obigen Formeln

L für Alkyl, Alkoxy, Halogen, COOR, OCOR, CONHR oder NHCOR steht,
X für S, O, N, $CH_2$ oder eine Einfachbindung steht,
A eine Einfachbindung, $(CH_2)_n$, $O(CH_2)_n$, $S(CH_2)_n$, $NR(CH_2)_n$,

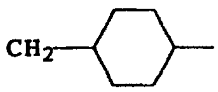, 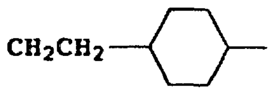, 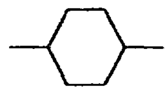, 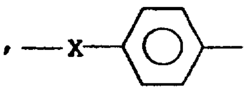

oder

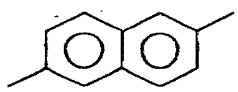

bedeutet,

R Alkyl oder Wasserstoff bedeutet,
$R^1$ Wasserstoff, Halogen, Alkyl oder Phenyl bedeutet und
n eine ganze Zahl von 1 bis 15 bedeutet.

Bevorzugt sind Polyester, die mindestens eine Dicarbonsäureeinheit der Formel

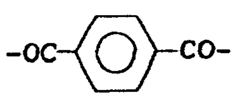, 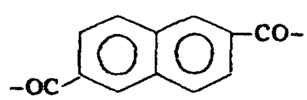

oder

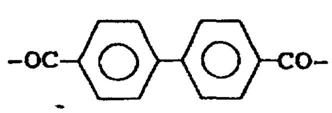,

und mindestens eine Dioleinheit der Formel

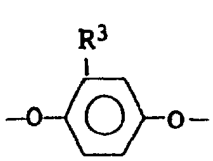, 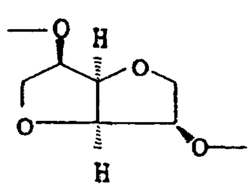 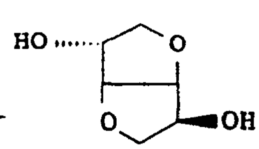,

wobei $R^3$ für H, Halogen, $C_1$-$C_4$-Alkyl, insbesondere $CH_3$ oder $C(CH_3)_3$, oder Phenyl steht, enthalten.

Weitere bevorzugte Verbindungen sind Diester der Formel P-Y-B-CO-O-A-O-CO-B-Y-P, worin P für eine Propargylendgruppe der oben definierten Formel steht, Y für O, S oder $NR^2$ ($R^2$ = $C_1$-$C_4$-Alkyl) steht, B für

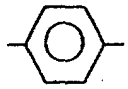 , 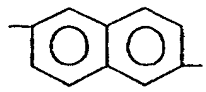 oder 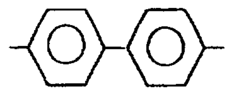

steht, wobei jede Phenylgruppe oder die Naphthylgruppe 1, 2 oder 3 Substituenten aufweisen kann, die unabhängig voneinander ausgewählt sind unter $C_1$-$C_4$-Alkyl, $C_1$-$C_4$-Alkoxy, Halogen oder Phenyl, und A (zusammen mit den benachbarten Sauerstoffatomen) für eine der oben genannten Dioleinheiten steht.

Besonders bevorzugte Diester sind solche der oben genannten Formel, in der B für

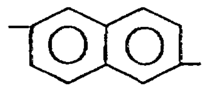 oder 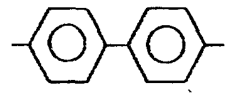

steht, und insbesondere Diester der Formel

$$HC{\equiv}CCH_2O\text{-}B\text{-}CO\text{-}O\text{-}A\text{-}O\text{-}CO\text{-}B\text{-}OCH_2\text{-}C{\equiv}CH,$$

worin

(XI) B für

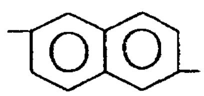

und

A für

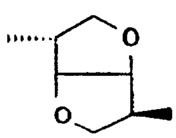 , 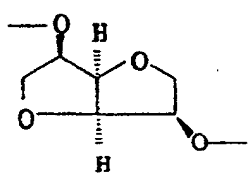 , 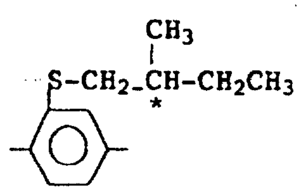 ,

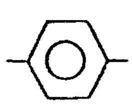 , 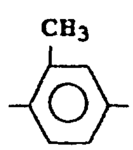 , 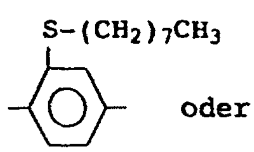 oder 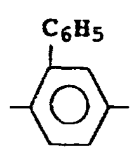

steht, oder

(XII) B für

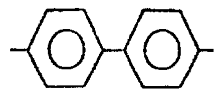

steht, und

A die unter XI genannten Bedeutungen besitzt.

Weitere bevorzugte Verbindungen sind Polycarbonate, die mindestens eine Dioleinheit der oben genannten Formeln,
insbesondere der Formeln

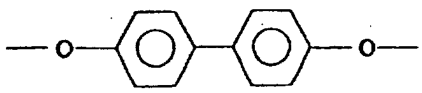 , 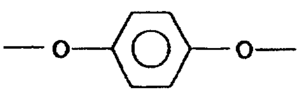 , 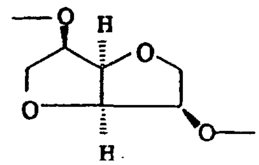 ,

oder

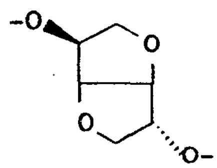

eingebaut enthalten.

Bevorzugte Polycarbonate sind dabei solche, die als Dioleinheiten mindestens eine mesogene Einheit der Formel

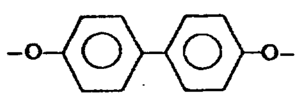 oder 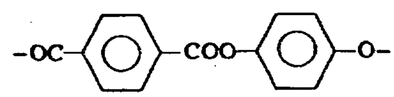

und mindestens eine chirale Einheit der Formel

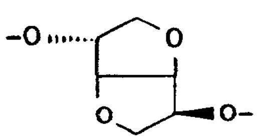 oder 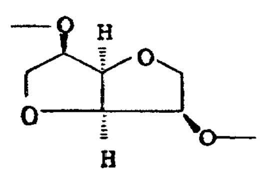

und gegebenenfalls eine nicht-chirale Einheit der Formel

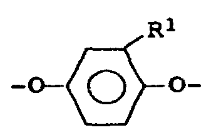

enthalten, wobei $R^1$ die oben angegebenen Bedeutungen besitzt und insbesondere für H oder $CH_3$ steht.

Besonders bevorzugte Polycarbonate sind solche mit Propargylendgruppen der Formel $HC{\equiv}CCH_2O\text{-}R^5\text{-}CO$, worin $R^5$ für

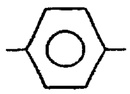 oder 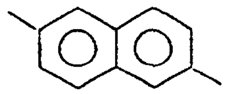 steht.

Als Polymere der Gruppe c) sind außerdem cholesterische Polycarbonate, die auch an nicht-terminaler Position photoreaktive Gruppen enthalten, geeignet. Solche Polycarbonate werden in der DE-A-196 31 658 beschrieben. Sie entsprechen vorzugsweise der Formel XIII

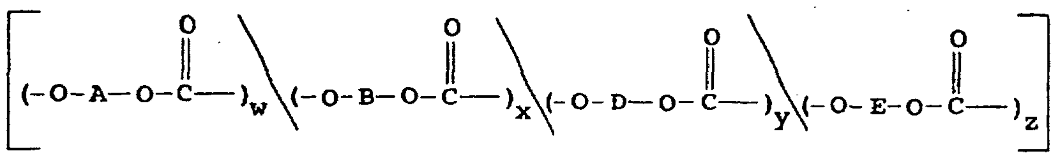 ,

(XIII)

wobei das Molverhältnis w/x/y/z etwa 1 bis 20/etwa 1 bis 5/etwa 0 bis 10/etwa 0 bis 10 beträgt. Besonders bevorzugt ist ein Molverhältnis w/x/y/z von etwa 1 bis 5/etwa 1 bis 2/etwa 0 bis 5/etwa 0 bis 5.

In der Formel XIII steht

A für eine mesogene Gruppe der Formel

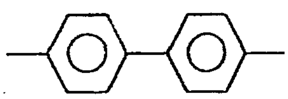 oder 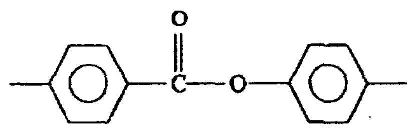

B für eine chirale Gruppe der Formel

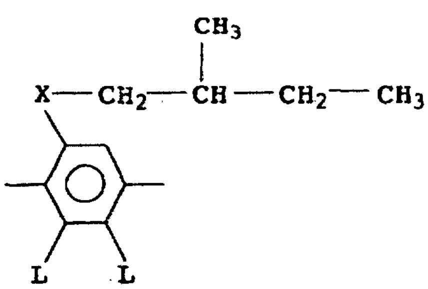 , 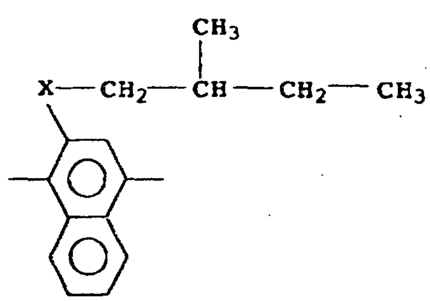 ,

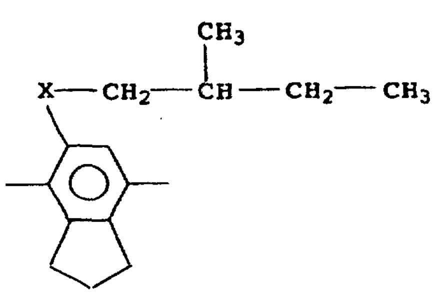 , 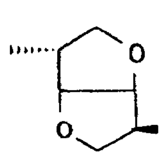 (Isosorbid) , 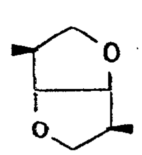 (Isomannid), 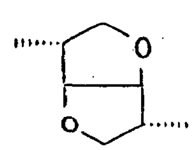 (Isoidid),

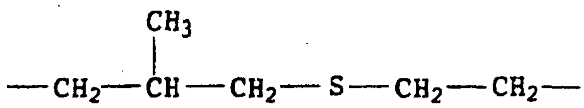

,

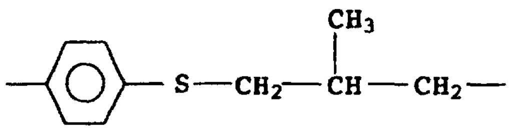

,

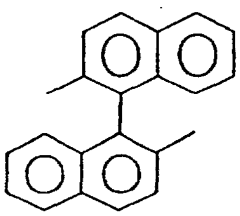

oder

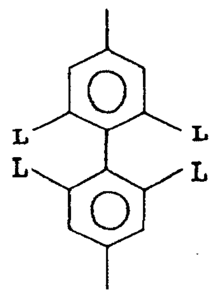

,

D für eine photoreaktive Gruppe der Formel

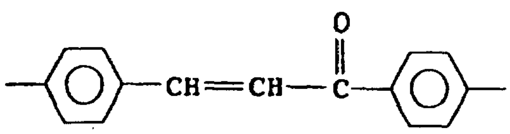

oder

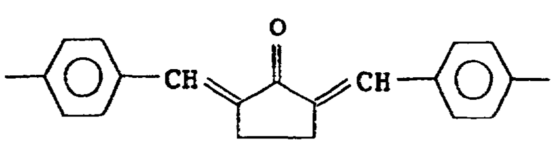

und

E für eine weitere, nicht-chirale Gruppe der Formel

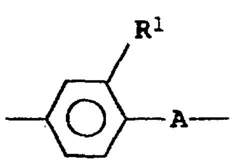

,

wobei in den obigen Formeln

L für Alkyl, Alkoxy, Halogen, COOR, OCOR, CONHR oder NHCOR steht,

X für S, O, N, $CH_2$ oder eine Einfachbindung steht,

R für Alkyl oder Wasserstoff steht,

A eine Einfachbindung, $(CH_2)_n$, $O(CH_2)_n$, $S(Cu_2)_n$, $NR(CH_2)_n$,

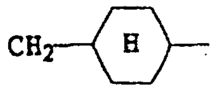 , 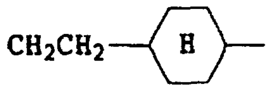 , 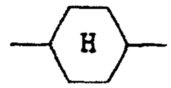 , 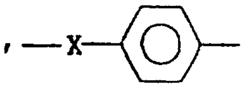

oder

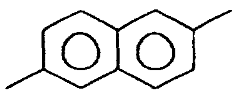

bedeutet,

$R^1$ Wasserstoff, Halogen, Alkyl oder Phenyl bedeutet und

n eine ganze Zahl von 1 bis 15 bedeutet.

Wenn $R^1$ für Alkyl, Halogen und A für eine Einfachbindung stehen oder wenn $R^1$ für H oder Alkyl und A für

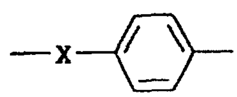 ,

$—O(CH_2)_n—$ , $—S(CH_2)_n$ oder $—NR(CH_2)_n$
steht, handelt es sich um löslichkeitsverbessernde Gruppen. Beispiele hierfür sind

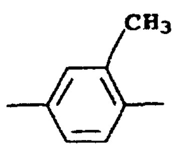 , 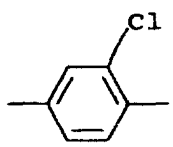 oder 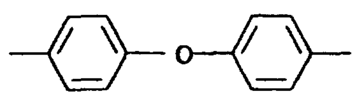 .

Isosorbid, Isomannid und/oder Isoidid ist die bevorzugte chirale Komponente.

Der Anteil der chiralen Diolstruktureinheiten liegt bevorzugt im Bereich von 1 bis 80 mol-% des Gesamtgehaltes an Diolstruktureinheiten, besonders bevorzugt 2 bis 20 mol-%, je nach gewünschtem Interferenzfarbton.

Geeignete Polymere der Gruppe e) sind chiral nematische Polyester mit flexiblen Ketten, die Isosorbid-, Isomannid- und/oder Isoidideinheiten, vorzugsweise Isosorbideinheiten, umfassen und zur Flexibilisierung der Ketten mindestens eine Einheit enthalten, die ausgewählt ist unter (und abgeleitet von)

(a) aliphatischen Dicarbonsäuren,
(b) aromatischen Dicarbonsäuren mit flexiblem Spacer,
(c) $\alpha,\omega$-Alkanoiden,
(d) Diphenolen mit flexiblem Spacer und
(e) Kondensationsprodukten aus einem Polyalkylenterephthalat oder Polyalkylennaphthalat mit einem acylierten Diphenol und einem acylierten Isosorbid,

wie sie in der DE-A-197 04 506 beschrieben werden.

Die Polyester sind nicht kristallin und bilden stabile Grandjean-Texturen aus, die sich beim Abkühlen unter die Glasübergangstemperatur einfrieren lassen. Die Glasübergangstemperaturen der Polyester wiederum liegen trotz der Flexibilisierung oberhalb von 80 °C, vorzugsweise oberhalb von 90 °C, insbesondere oberhalb von 100 °C.

Die einsetzbaren Polyester enthalten als Einheiten (a) vorzugsweise solche der Formel

$$-OC-(CH_2)_n-CO-$$

wobei n für eine Zahl im Bereich von 3 bis 15, insbesondere 4 bis 12 steht und besonders bevorzugt Adipinsäure; als Einheiten (b) vorzugsweise solche der Formel

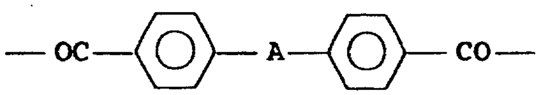 ,

wobei

A für $(CH_2)_n$, $O(CH_2)_nO$ oder $(CH_2)_o$-O-$(CH_2)_p$ steht,

n für eine Zahl im Bereich von 3 bis 15, insbesondere 4 bis 12, besonders bevorzugt 4 bis 10 steht und

o und p unabhängig voneinander für eine Zahl im Bereich von 1 bis 7 stehen;

als Einheiten (c) vorzugsweise solche der Formel

$$-O-(CH_2)_n-O-$$

oder

$$-O-(CH_2-CH_2-O)_m- ,$$

wobei

n für eine Zahl im Bereich von 3 bis 15, insbesondere 4 bis 12, besonders bevorzugt 4 bis 10 steht und
m für eine Zahl im Bereich von 1 bis 10 steht; und

als Einheiten (d) vorzugsweise solche der Formel

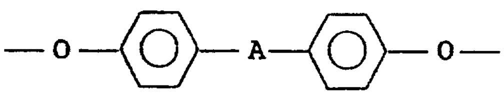 ,

wobei

A für $(CH_2)_n$, $O(CH_2)_nO$ oder $(CH_2)_o$-O-$(CH_2)_p$ steht,

n für eine zahl im Bereich von 3 bis 15, insbesondere 4 bis 12, besonders bevorzugt 4 bis 10 steht und

o und p unabhängig voneinander für eine Zahl im Bereich von 1 bis 7 stehen.

Die einsetzbaren Polyester enthalten außerdem als nicht flexible Säurekomponente vorzugsweise Dicarbonsäureeinheiten der Formel

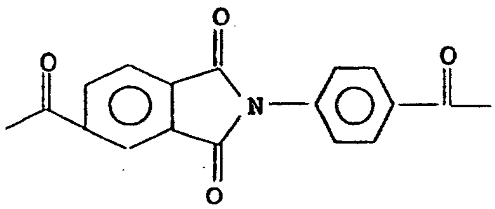 , 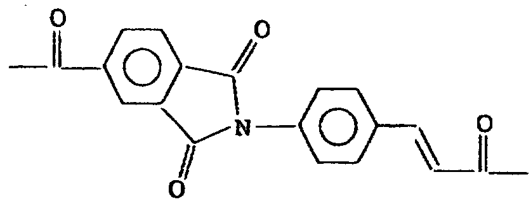 ,

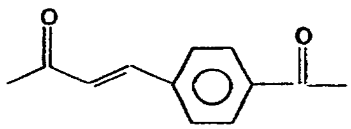 , 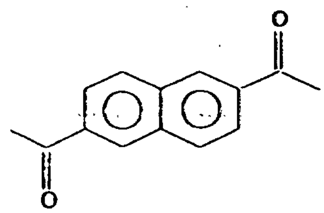 ,

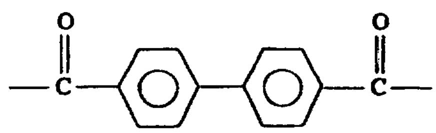 , 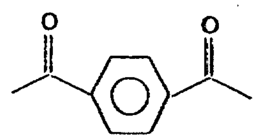 ,

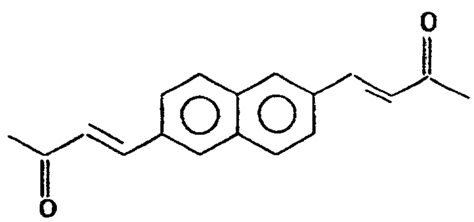 ,

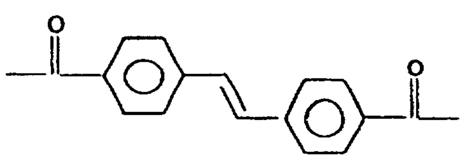 oder 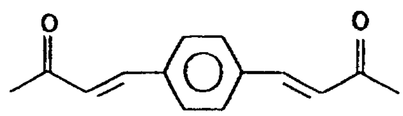

und als nicht flexible Alkoholkomponente Dioleinheiten der Formel

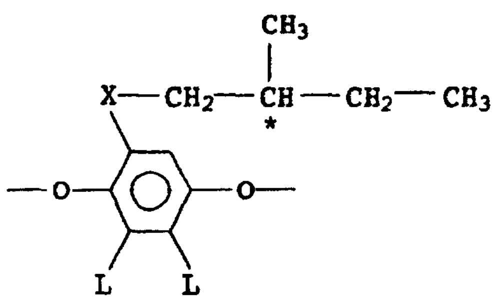 , 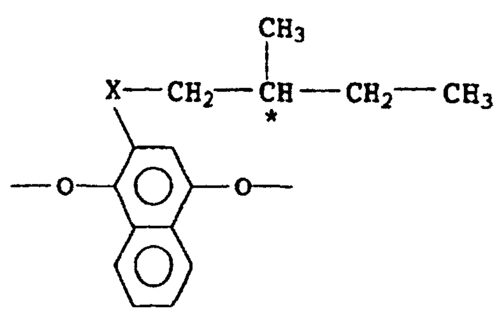 ,

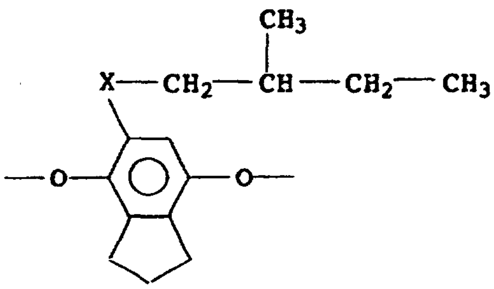 , 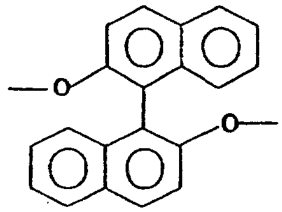 ,

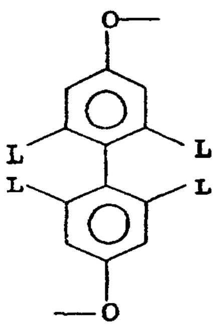 , 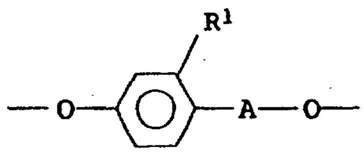 ,

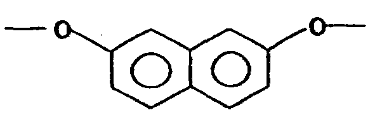 oder 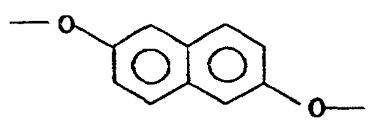

wobei in den obigen Formeln

L für Alkyl, Alkoxy, Halogen, COOR, OCOR, CONHR oder NHCOR steht,

X für S, O, N, $CH_2$ oder eine Einfachbindung steht,

A eine Einfachbindung

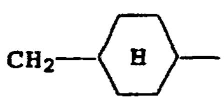 , 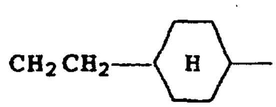 , 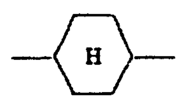 ,

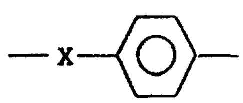 oder 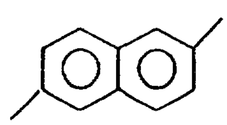

bedeutet und
wobei

$R^1$ Wasserstoff, Halogen, Alkyl oder Phenyl bedeutet und

R Alkyl oder Wasserstoff bedeutet.

Gegebenenfalls enthalten die einsetzbaren Polyester zusätzliche flexible Dioleinheiten der Formel

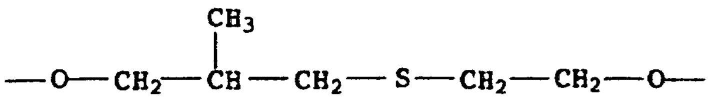 ,

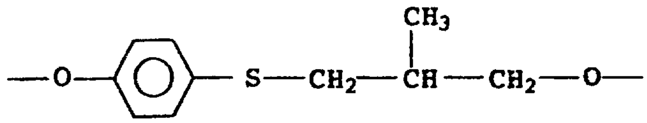 ,

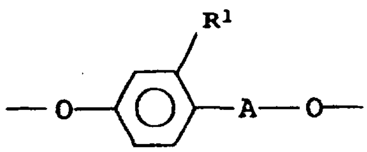 ,

wobei

$R^1$ Wasserstoff, Halogen, Alkyl oder Phenyl bedeutet,

A $(CH_2)n$, $O(CH_2)_n$, $S(CH_2)_n$ oder $NR(CH_2)_n$ bedeutet und

n eine Zahl von 1 bis 15 bedeutet.

Bevorzugte Polymere der Gruppe d) sind beispielsweise vernetzbare, cholesterische Copolyisocyanate, wie sie in der US-A-08 834 745 beschrieben werden. Solche Copolyisocyanate weisen wiederkehrende Einheiten der Formeln

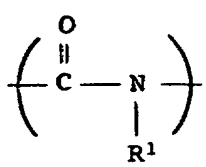 (III),

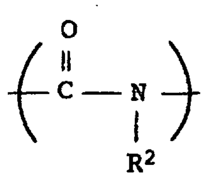 (IV)

und gegebenenfalls der Formel

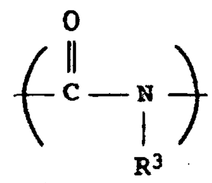 (V)

auf ,
worin

$R^1$ für einen chiralen aliphatischen oder aromatischen Rest steht,

$R^2$ für einen vernetzbaren Rest steht und

$R^3$ für einen achiralen Rest steht.

Soweit nicht anders angegeben, ist hier unter "Alkyl" (auch in Bedeutungen wie Alkoxy, Dialkyl, Alkylthio etc.) ein verzweigtes und unverzweigtes $C_1$-$C_{12}$-Alkyl, vorzugsweise $C_3$-$C_{12}$-, besonders bevorzugt $C_4$-$C_{10}$-, insbesondere $C_6$-$C_{10}$-Alkyl zu verstehen.
vorzugsweise ist $R^1$ ausgewählt unter (chiralen) verzweigten oder unverzweigten Alkyl-, Alkoxyalkyl-, Alkylthioalkyl-, Cycloalkyl-, Alkylphenyl- oder $C_3$-$C_9$-Epoxyalkylresten oder Resten von Estern von $C_1$-$C_6$-Fettsäuren mit $C_1$-$C_6$-Alkanolen oder $C_3$-$C_9$-Dialkylketonen. Der Esterrest kann sowohl über den Fettsäureanteil als auch über den Alkanolrest an das N-Atom gebunden sein. Der Rest $R^1$ kann 1, 2 oder 3 Substituenten aufweisen, die gleich oder verschieden und ausgewählt sind unter Alkoxygruppen, Di-$C_1$-$C_4$-alkylaminogruppen, CN, Halogenatomen oder $C_1$-$C_4$-Alkylthiogruppen.
Vorzugsweise ist $R^1$ ausgewählt unter Alkyl, Alkoxyalkyl, Resten von Estern von $C_1$-$C_6$-Fettsäuren mit $C_1$-$C_6$-Alkanolen, $C_3$-$C_9$-Dialkylketonen und epoxidierten $C_3$-$C_9$-Epoxyalkylresten, wobei $R^1$ durch 1 oder 2 Reste substituiert sein kann, die gleich oder verschieden und ausgewählt sind unter Alkoxy, Halogen, CN oder $CF_3$. Bevorzugte Substituenten für verzweigte oder unverzweigte Alkyl- oder Alkoxyreste sind ausgewählt unter Alkoxygruppen, Halogenatomen oder CN; für Ester von $C_1$-$C_6$-Fettsäuren mit $C_1$-$C_6$-Alkanolen unter Alkoxygruppen, Halogenatomen, CN oder $CF_3$ und für $C_3$-$C_9$-Dialkylketone unter Alkoxygruppen, Halogenatomen oder CN.
Insbesondere weist die Hauptkette des Restes $R^1$ eine Länge von 3 bis 12, insbesondere 6 bis 10, vorzugsweise 6 bis 8 Gliedern (C-, O- und/oder S-Atome) auf. Besonders bevorzugt sind Reste $R^1$, die ausgewählt sind unter

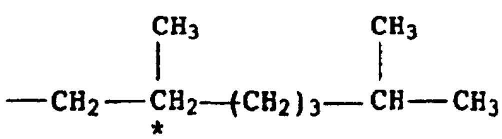

2,6-Dimethylheptyl

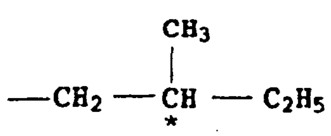 ,

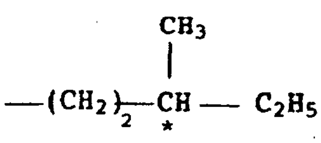 ,

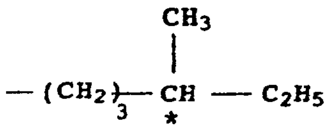 , 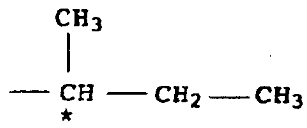 ,

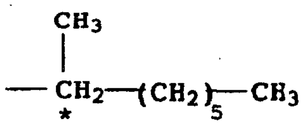 , 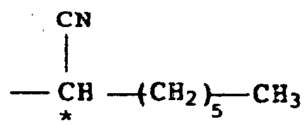 ,

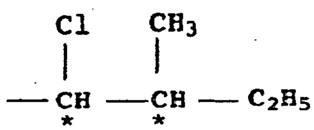 , 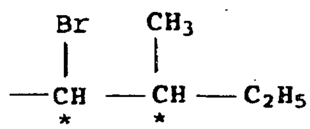 ,

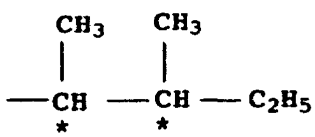 , 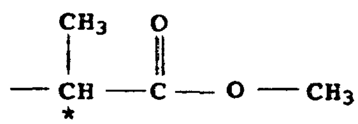 ,

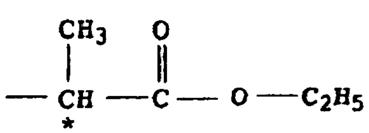 , 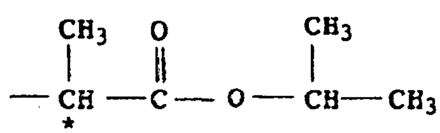 ,

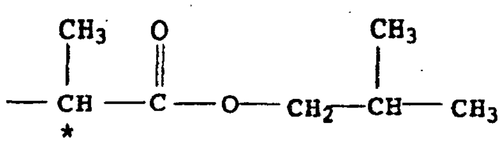 , 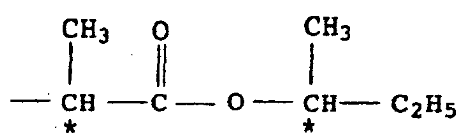 ,

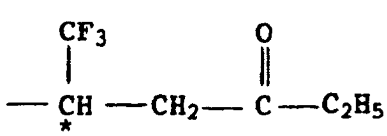 , 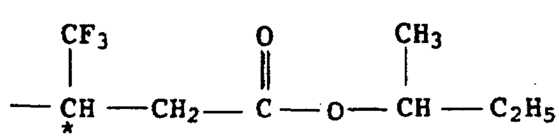 ,

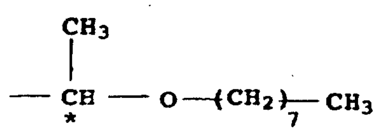 , 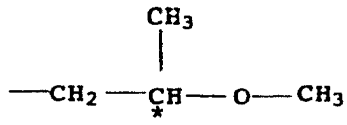 ,

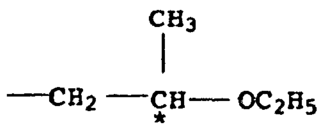 , 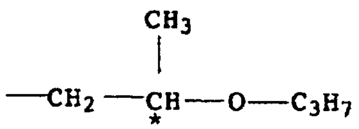 ,

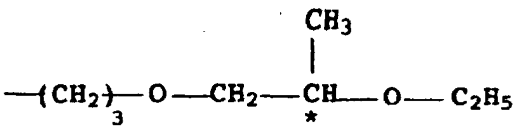 , 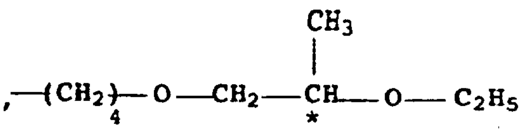 ,

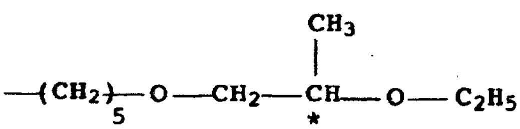 , 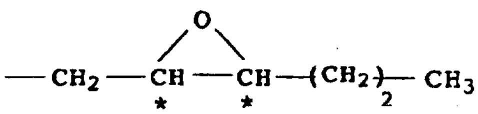 ,

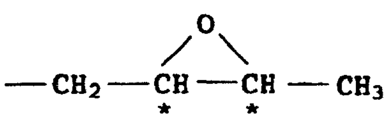 und 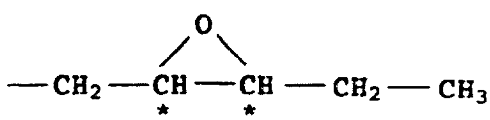

Ganz besonders bevorzugt ist die Komponente III der einsetzbaren Copolyisocyanate von 2,6-Dimethylheptylisocyanat abgeleitet.

Der Rest $R^2$ der einsetzbaren Copolyisocyanate ist vorzugsweise ausgewählt unter $C_3$-$C_{11}$-Alkenylresten, $C_4$-$C_{11}$-Vinyletherresten (= Vinyl-$C_2$-$C_9$-Alkylethern), ethylenisch ungesättigten $C_3$-$C_{11}$-Carbonsäureresten und Estern von ethylenisch ungesättigten $C_3$-$C_6$-Monocarbonsäuren mit $C_2$-$C_6$-Alkanolen, wobei die Bindung an das N-Atom über den Alkanolrest des Esters erfolgt. Besonders bevorzugt ist der Rest ausgewählt unter Methylacrylat, Ethylacrylat, Propylacrylat, n-Butylacrylat, Isobutylacrylat, 2-Ethylhexylacrylat, Methylmethacrylat, Ethylmethacrylat, Propylmethacrylat, n-Butylmethacrylat, Isobutylmethacrylat, 2-Ethylhexylmethacrylat, insbesondere unter Ethylacrylat oder Ethylmethacrylat.

Der Rest $R^3$ besitzt vorzugsweise die gleichen Bedeutungen wie der Rest $R^1$. Er ist aber achiral, d. h. er weist kein Chiralitätszentrum auf oder liegt als racemisches Gemisch vor.

Besonders bevorzugt weist die Hauptkette des Restes $R^3$ eine Länge von 4 bis 12, insbesondere 6 bis 10, vorzugsweise 6 bis 8 Gliedern (C-, O- und/oder S-Atome) auf. Ganz besonders bevorzugt ist die Komponente V der erfindungsgemäßen Copolyisocyanate von n-Hexylisocyanat, n-Heptylisocyanat oder n-Octylisocyanat abgeleitet.

Die Komponenten III, IV und V sind vorzugsweise im Molmengenverhältnis III:IV:V von etwa 1 bis 20 : 1 bis 20 : 50 bis 98, insbesondere etwa 5 bis 15 : 5 bis 15 : 65 bis 90, besonders bevorzugtermaßen etwa 15:10:75 vorhanden.

Die Einheiten III, IV und V können in den einsetzbaren Copolyisocyanaten statistisch verteilt sein.

Erfindungsgemäß ganz besonders bevorzugt enthalten $A^1$ und $A^2$ chirale Verbindungen und nematische Monomere gemäß Gruppe b), insbesondere chirale Verbindungen der Formel 2:

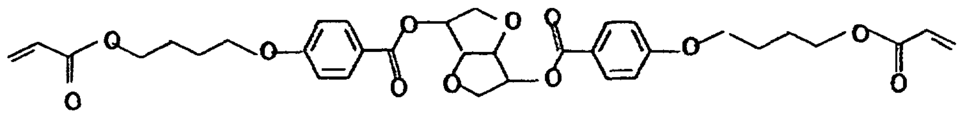

(2)

oder der Formel 5:

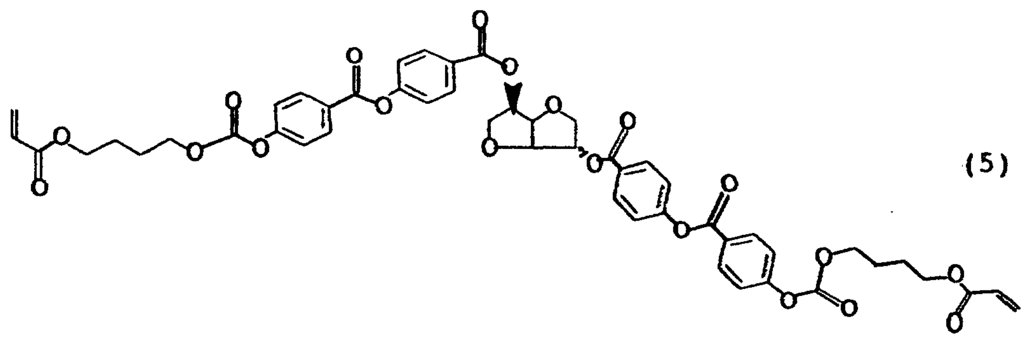

(5)

und nematische Monomere der Formel 1:

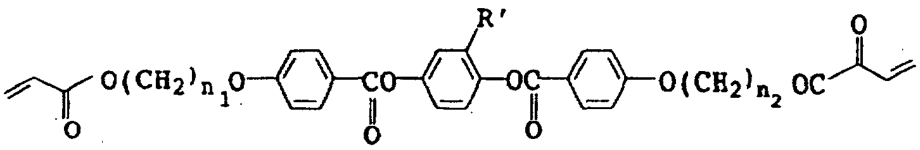

(1)

oder bevorzugt der Formel 3:

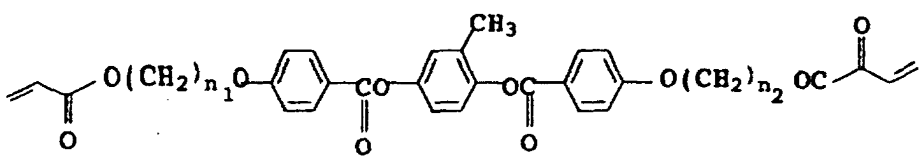

(3)

oder besonders bevorzugt der Formel 4:

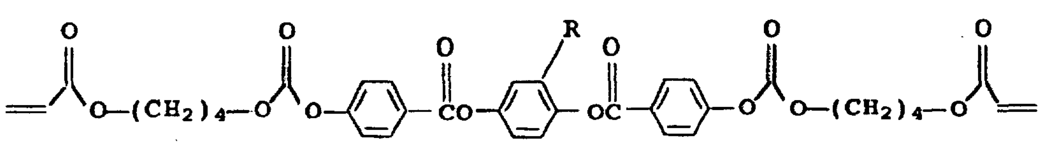

(4)

in gehärtetem zustand, wobei in den Formeln 1 und 3 $n_1$ und $n_2$ unabhängig voneinander für 4 oder 6 stehen, R' in Formel 1 für H oder Cl steht und die Monomeren der Formel 1 oder 3 vorzugsweise als Gemische von Verbindungen mit $n_1/n_2$ = 4/4, 4/6, 6/4 oder 6/6 eingesetzt werden, und R in Formel 4 für H, Cl oder $CH_3$ steht. Erfindungsgemäß können jedoch auch andere cholesterische Gemische, beispielsweise die in der EP-A-686 674 offenbarten Gemische, in gehärtetem Zustand in $A^1$ und $A^2$ enthalten sein.

Die cholesterischen Gemische bzw. die Absorptionspigment enthaltenden Formulierungen können mit jedem geeigneten Verdünnungsmittel vor dem Aufbringen auf den Träger verdünnt werden.

In dem erfindungsgemäßen Verfahren einsetzbare Verdünnungsmittel sind für die Verbindungen der Gruppen a) oder b) lineare oder verzweigte Ester, besonders Essigsäureester, cyclische Ether und Ester, Alkohole, Lactone, aliphatische und aromatische Kohlenwasserstoffe, wie Toluol, Xylol und Cyclohexan, sowie Ketone, Amide, N-Alkylpyrrolidone, besonders N-Methylpyrrolidon, und insbesondere Tetrahydrofuran (THF), Dioxan und Methylethylketon (MEK).

Geeignete Verdünnungsmittel für die Polymere der Gruppe c) sind beispielsweise Ether und cyclische Ether wie Tetrahydrofuran oder Dioxan, chlorierte Kohlenwasserstoffe, wie Dichlormethan, 1,1,2,2-Tetrachlorethan, 1-Chlornaphthalin, Chlorbenzol oder 1,2-Dichlorbenzol. Diese Verdünnungsmittel sind besonders für Polyester und Polycarbonate geeignet. Geeignete Verdünnungsmittel für Cellulosederivate sind beispielsweise Ether, wie Dioxan, oder Ketone, wie Aceton. Werden Copolyisocyanate als Polymere der Gruppe d) eingesetzt, ist es sinnvoll, polymerisierbare Verdünnungsmittel, wie in der US-A-08 834 745 beschrieben, zu verwenden. Solche polymerisierbaren Verdünnungsmittel sind beispielsweise

- Ester $\alpha,\beta$-ungesättigter Mono- oder Dicarbonsäuren, insbesondere $C_3$-$C_6$-Mono- oder Dicarbonsäuren, mit $C_1$-$C_{12}$-Alkanolen, $C_2$-$C_{12}$-Alkandiolen oder deren $C_1$-$C_6$-Alkylether und Phenylether, beispielsweise Acrylate und Methacrylate, Hydroxyethyl- oder Hydroxypropylacrylat oder -methacrylat sowie 2-Ethoxyethylacrylat oder -methacrylat;
- Vinyl-$C_1$-$C_{12}$-alkylether, wie Vinylethyl-, Vinylhexyl- oder Vinyloctylether;
- Vinylester von $C_1$-$C_{12}$-Carbonsäuren, wie Vinylacetat, Vinylpropionat, Vinyllaurat;
- $C_3$-$C_9$-Epoxide, wie 1,2-Butylenoxid, Styroloxid;
- N-Vinylpyrrolidon, N-Vinylcaprolactam, N-Vinylformamid;
- vinylaromatische Verbindungen, wie Styrol, $\alpha$-Methylstyrol, Chlorstyrol, und
- Verbindungen mit zwei oder mehreren vernetzbaren Gruppen, wie Diester von Diolen (einschließlich Polyethylenglykole) mit Acryl- oder Methacrylsäure oder Divinylbenzol.

Beispiele für bevorzugte polymerisierbare Verdünnungsmittel sind 2-Ethoxyethylacrylat, Diethylenglykoldiacrylat, Ethylenglykoldimethacrylat, Diethylenglykoldimethacrylat, Triethylenglykoldimethacrylat, Diethylenglykolmonomethyletheracrylat, Phenoxyethylacrylat und Tetraethylenglykoldimethacrylat. Ein besonders bevorzugtes polymerisierbares Verdünnungsmittel ist Styrol.

Auch die Gemische der Gruppen a), b) oder c) können in kleinen Mengen polymerisierbare Verdünnungsmittel zusätzlich zu dem inerten Verdünnungsmittel enthalten. Bevorzugte, a), b) oder c) zusetzbare, polymerisierbare Lösungsmittel sind Acrylate, insbesondere höherfunktionelle Acrylate wie Bis, Tris- oder Tetraacrylate, besonders bevorzugt hochsiedende Oligoacrylate. Die bevorzugte Zusatmenge liegt bei etwa 5 Gew.-%, bezogen auf das Gesamtgewicht des Gemischs.

Gegebenenfalls kann auch Wasser dem Verdünnungsmittel zugesetzt sein oder sogar als alleiniges Verdünnungsmittel eingesetzt werden.

Das cholesterische Gemisch kann für die photochemische Polymerisation handelsübliche Photoinitiatoren enthalten. Für eine Härtung durch Elektronenstrahlen sind solche nicht notwendig. Geeignete Photoinitiatoren sind beispielsweise Isobutyl-benzoinether, 2,4,6-Trimethylbenzoyldiphenylphosphinoxid, 1-Hydroxycyclohexylphenylketon, 2-Benzyl-2-dimethylamino-1-(4-morpholinophenyl)-furan-1-on, Mischungen von Benzophenon und 1-Hydroxycyclohexyl-phenylketon, 2,2-Dimethoxy-2-phenylacetophenon, perfluorierte Diphenyltitanocene, 2-Methyl-1-(4-[methylthio] phenyl)-2-(4-morpholinyl)-1-propanon, 2-Hydroxy-2-methyl-1-phenylpropan-1-on, 4-(2-Hydroxyethoxy)phenyl-2-hydroxy-2-propylketon, 2,2-Diethoxyacetophenon, 4-Benzoyl-4'-methyldiphenyl-sulfid, Ethyl-4-(dimethylamino) benzoat, Mischungen von 2-Isopropylthioxanthon und 4-Isopropyl-thioxanthon, 2-(Dimethylamino)ethylbenzoat, d, l-Campherchinon, Ethyl-d,l-campherchinon, Mischungen von Benzophenon und 4-Methylbenzophenon, Benzophenon, 4,4'-Bisdimethylamin-benzophenon, ($\eta^5$-Cyclopentadienyl) ($\eta^6$-isopropylphenyl)-eisen(II)-hexafluorophosphat, Triphenylsulfonium-hexafluorophosphat oder Mischungen von Triphenylsulfoniumsalzen, sowie Butandioldiacrylat, Dipropylenglykoldiacrylat, Hexandioldiacrylat, 4-(1,1-Dimethylethyl)cyclohexylacrylat, Trimethylolpropantriacrylat und Tripropylenglykoldiacrylat.

Die Brillanz der cholesterischen Schichten $A^1$ und $A^2$ kann durch Zugabe geringer Mengen geeigneter Verlaufsmittel gesteigert werden. Einsetzbar sind etwa 0,005 bis 1 Gew.-%, insbesondere 0,01 bis 0,5 Gew.-%, bezogen auf die Menge an eingesetztem Cholester. Geeignete Verlaufsmittel sind beispielsweise Glykole, Siliconöle und insbesondere Acrylatpolymere, wie die unter der Bezeichnung Byk 361 bzw. Byk 358 der Firma Byk-Chemie erhältlichen Acrylatcopolymere und die unter der Bezeichnung Tego flow ZFS 460 der Fa. Tego erhältlichen modifizierten silikonfreien Acrylatpolymere.

Gegebenenfalls enthält das cholesterische Gemisch auch Stabilisatoren gegen UV- und Wettereinflüsse. Hierfür eignen sich zum Beispiel Derivate des 2,4-Dihydroxybenzophenons, Derivate des 2-Cyan-3,3-diphenylacrylates, Derivate des 2,2',4,4'-Tetrahydroxybenzophenons, Derivate des Orthohydroxyphenylbenztriazols, Salicylsäureester, Orthohydroxyphenyl-S-triazine oder sterisch gehinderte Amine. Diese Stoffe können allein oder vorzugsweise in Form von Gemischen eingesetzt werden.

Ein weiterer Gegenstand der vorliegenden Erfindung ist ein Verfahren zur Herstellung eines erfindungsgemäßen Pigments, das dadurch gekennzeichnet ist, dass man die Schichten $A^1$, B und $A^2$ gleichzeitig oder zeitlich versetzt übereinander auf einen Träger mittels Gießbeschichtung aufbringt, gleichzeitig oder zeitlich versetzt thermisch, durch UV-Strahlen, Elektronenstrahlen oder durch schnelles Abkühlen unter die Glasübergangstemperatur härtet, die ausgehärteten Schichten vom Träger entfernt und anschließend zu Pigmenten zerkleinert.

Der Träger ist vorzugsweise beweglich, besonders bevorzugt beweglich und bandförmig.

Als Schichtträger dienen vorzugsweise bekannte Filme aus Polyestern, wie Polyethylenterephthalat oder Polyethylennaphthalat sowie Polyolefinen, Cellulosetriacetat, Polycarbonaten, Polyamiden, Polyimiden, Polyamidoimiden, Polysulfonen, Aramiden oder aromatischen Polyamiden. Die Dicke der Schichtträger beträgt vorzugsweise etwa 5 bis 100 µm, insbesondere etwa 10 bis 20 µm. Der Schichtträger kann vorher einer Koronaentladungsbehandlung, einer Plasmabehandlung, einer leichten Adhäsionsbehandlung, einer Wärmebehandlung, einer Staubentfernungsbehandlung oder ähnlichem unterworfen werden. Der Schichtträger weist vorzugsweise eine mittlere Mittellinien-Oberflächenrauhigkeit von 0,03 µm oder weniger, insbesondere von 0,02 µm oder weniger, besonders bevorzugt von 0,01 µm oder weniger auf. Außerdem ist es erwünscht, dass der Träger nicht nur eine derart geringe mittlere Mittellinien-Oberflächenrauhigkeit aufweist, sondern auch keine großen Vorsprünge (Erhebungen) von 1 µm oder mehr besitzt. Das Rauhigkeitsprofil der Oberfläche des Trägers kann durch Füllstoffe, die dem Schichtträger bei dessen Herstellung zugesetzt werden, variiert werden.

Beispiele für geeignete Füllstoffe sind Oxide und Carbonate von Ca, Si und Ti sowie organische feine Pulver von Acrylsubstanzen.

Der Träger kann auch eine metallisierte Folie oder ein vorzugsweise poliertes Metallband sein.

Die Schichten $A^1$, B und $A^2$ können niedrig- oder hochviskos, vorzugsweise jedoch niedrigviskos auf den Träger aufgebracht werden. Zu diesem Zweck können die cholesterischen Gemische bzw. die Absorptionspigment enthaltenden Formulierungen unverdünnt oder niedrigverdünnt bei erhöhter Temperatur oder hochverdünnt bei niedriger Temperatur auf den Träger aufgebracht werden. Es ist besonders bevorzugt, die drei Schichten $A^1$, B und $A^2$ in einem Auftragungsvorgang nass-in-nass auf den Träger aufzubringen, gegebenenfalls gemeinsam zu trocknen und danach gemeinsam zu härten.

Für den gleichzeitigen Auftrag nass-in-nass der genannten Schichten ist es besonders bevorzugt, wenn die Schicht B Absorptionspigmente in einer Matrix aus dem gleichen cholesterischen Gemisch eingebunden enthält, das auch in den Schichten $A^1$ und $A^2$ enthalten ist. Hierdurch werden möglicherweise störende Schichtgrenzen zwischen $A^1$, B und $A^2$ vermieden, so dass ein homogenes System erhalten wird, das im mittleren Bereich homogen verteilte Pigmente enthält.

Für das gleichzeitige Auftragen der genannten Schichten sind Gießverfahren besonders geeignet, insbesondere Messer- bzw. Rakelgießverfahren, Extrusions- bzw. Abstreifgießverfahren sowie das Kaskadengießverfahren.

Beim Messer- bzw. Rakelgießverfahren erfolgt das Auftragen der Flüssigkeit auf einen Träger durch einen Schlitz eines Gießerblocks, wobei die Schichtdicke über einen definierten Rakelspalt zwischen einer Walze, über die der Träger geführt wird und der Gießerkante eingestellt werden kann. Zum Auftragen der untersten (ersten) Schicht wird der erste Gießerblock gegen die Walze verstellt, zum Auftragen der zweiten Schicht wird ein zweiter Gießerblock gegen den ersten Gießerblock verstellt und zum Auftragen der dritten Schicht wird ein dritter Gießerblock gegen den zweiten verstellt. Ein analoges Verfahren wird in der DE-A-19 504 930 beschrieben, worauf hiermit ausdrücklich Bezug genommen wird. Alle drei Flüssigkeiten laufen ihrem jeweiligen Gießrakel zu und werden gleichzeitig übereinander abgerakelt.

Beim Extrusions- bzw. Abstreifgießverfahren wird ein flexibler Träger, beispielsweise eine Folie, mit definierter Bahnspannung zwischen zwei Walzen an dem Gießerkopf vorbei geführt. Aus drei parallelen, quer zur Bahnlaufrichtung angeordneten Gießschlitzen werden die der erwünschten Schichtstärke angepassten Flüssigkeitsmengen gleichzeitig auf das Substrat aufgebracht. Ein solches Verfahren ist beispielsweise in der EP-A-431 630, der DE-A-3 733 031 und der EP-A-452 959 beschrieben, worauf hiermit ausdrücklich Bezug genommen wird.

Beim Kaskadengießverfahren wird der Träger über eine Walze geführt. Die aufzubringenden Flüssigkeiten laufen aus verschiedenartig angeordneten Schlitzen übereinander und dann gemeinsam auf den Träger auf. Dieses Verfahren ist gleichfalls in der DE-A-19 504 930 beschrieben.

Selbstverständlich ist es auch möglich, zunächst nur eine cholesterische Schicht aufzubringen, diese gegebenenfalls zu trocknen und zu härten und dann zwei Schichten nass-in-nass beispielsweise mit einem der oben aufgeführten Verfahren auf die gehärtete cholesterische Schicht aufzutragen. Ebenso kann jede Schicht einzeln nacheinander aufgetragen, gegebenenfalls getrocknet und gehärtet werden.

Bei Einsetz des Gießverfahren weist das gießfähige, cholesterische Gemisch vorzugsweise eine Viskosität im Bereich von etwa 10 bis 500 mPas, insbesondere etwa 10 bis 100 mPas, gemessen bei 23 °C, auf. Besonders bevorzugt bringt man das cholesterische Gemisch mit einer Geschwindigkeit von etwa 1 bis 800 m/min, insbesondere etwa 5 bis 100 m/min, auf den Träger auf. Vorzugsweise wird eine Gießvorrichtung verwendet, deren Gießspaltbreite im Bereich von etwa 2 bis 50 µm, insbesondere etwa 4 bis 15 µm liegt. Vorzugsweise erfolgt der Auftrag des cholesterischen Gemischs unter erhöhtem Druck, insbesondere bei einem Gießerüberdruck im Bereich von etwa 0,01 bis 0,7 bar, besonders bevorzugt 0,05 bis 0,3 bar.

Das Entfernen der ausgehärteten Schichten vom Träger kann zum Beispiel dadurch erfolgen, dass dieser über eine Umlenkrolle mit kleinem Durchmesser geführt wird. Infolgedessen blättert das vernetzte Material dann von dem Träger ab. Weitere bekannte Methoden sind gleichfalls geeignet, zum Beispiel das Abziehen des Trägers über eine scharfe Kante, Air Knife (Luftrakel), Ultraschall oder Kombinationen davon. Das nun trägerlose cholesterische Material wird auf eine gewünschte Korngröße zerkleinert. Dies kann beispielsweise durch Mahlen in Universalmühlen erfolgen. Die zerkleinerten Pigmente können anschließend zur Verengung der Korngrößenverteilung, beispielsweise durch einen Siebprozess, klassiert werden.

Ein weiterer Gegenstand der vorliegenden Erfindung sind Zusammensetzungen, die erfindungsgemäße Pigmente enthalten.

Besonders bevorzugte erfindungsgemäße Zusammensetzungen sind Überzugs- oder Beschichtungsmittel wie z. B. Farben und Lacke, die neben den erfindungsgemäßen Pigmenten eine oder mehrere Substanzen enthalten, die ausgewählt ist bzw. sind unter Wasserlakken, beispielsweise in Form von wässrigen Dispersionen, wie PMA, SA, Polyvinyl-Derivate, PVC, Polyvinylidenchlorid, SB-Copo, PV-AC-Copo-Harze, oder in Form von wasserlöslichen Bindemitteln, wie Schellack, Maleinharze, Kolophonium-modifizierte Phenolharze, lineare und verzweigte, gesättigte Polyester, Aminoplast-vernetzende, gesättigte Polyester, Fettsäure-modifizierte Alkydharze, plastifizierte Harnstoffharze, oder in Form von wasserverdünnbaren Bindemitteln, wie PUR-Dispersionen, EP-Harze, Harnstoffharze, Melaminharze, Phenolharze, Alkydharze, Alkydharzemulsionen, Siliconharzemulsionen; Pulverlacken, wie beispielsweise Pulverlacken für TRIBO/ES, wie Polyester-Beschichtungspulverharze, PUR-Beschichtungspulverharze, EP-Beschichtungspulverharze, EP/SP-Hybrid-Beschichtungspulverharze, PMA-Beschichtungspulverharze, oder Pulverlacke für Wirbelsintern, wie thermoplastifiziertes EPS, LD-PE, LLD-PE, HD-PE; lösemittelhaltigen Lacken, beispielsweise als Ein- und Zweikomponenten-Lacke (Bindemittel), wie Schellack, Kolophonium-Harzester, Maleinatharze, Nitrocellulosen, Kolophonium-modifizierte Phenolharze, physikalisch trocknende, gesättigte Polyester, Aminoplast-vernetzende, gesättigte Polyester, Isocyanat-vernetzende, gesättigte Polyester, selbstvernetzende, gesättigte Polyester, Alkyde mit gesättigten Fettsäuren, Leinölalkydharze, Sojaölhärze, Sonnenblumenölalkydharze, Safflorölalkydharze, Ricinenalkydharze, Holzöl-/Leinölalkydharze, Mischölalkydharze, harzmodifizierte Alkydharze, Styrol/Vinyltoluolmodifizierte Alkydharze, acrylierte Alkydharze, Urethan-modifizierte Alkydharze, Silicon-modifizierte Alkydharze, Epoxid-modifizierte Alkydharze, Isophthalsäure-Alkydharze, nichtplastifizierte Harnstoffharze, plastifizierte Harnstoffharze, Melaminharze, Polyvinylacetale, nichtvernetzende P(M)A-Homo- bzw. Copolymerisate, nichtvernetzende P(M)A-Homo- bzw. Copolymerisate mit Nichtacrylmonomeren, selbstvernetzende P(M)A-Homo- bzw. Copolymerisate, P(M)A-Copolymerisate mit anderen Nichtacrylmonomeren, fremdvernetzende P(M)A-Homo- bzw. Copolymerisate, fremdvernetzende P(M) A-Copolymerisate mit Nichtacrylmonomeren, Acrylat-Copolymerisationsharze, ungesättigte Kohlenwasserstoffharze, organisch lösliche Celluloseverbindungen, Silicon-Kombiharze, PUR-Harze, P-Harze, Peroxid-härtende, ungesättigte Kunstharze, strahlenhärtende Kunstharze, photoinitiatorhaltig, strahlenhärtende Kunstharze photoinitiatorfrei; lösemittelfreien Lacken (Bindemittel), wie Isocyanat-vernetzende, gesättigte Polyester, PUR-2K-Harzsysteme, PUR-1K-Harzsysteme feuchtigkeitshärtend, EP-Harze, sowie Kunstharze - einzeln oder in Kombination -, wie Acrylnitril-Butadien-Styrol-Copolymere, BS, Celluloseacetat, Celluloseacetobutyrat, Celluloseacetopropionat, Cellulosenitrat, Cellulosepropionat, Kunsthorn, Epoxidharze, Polyamid, Polycarbonat, Polyethylen, Polybutylenterephthalat, Polyethylenterephthalat, Polymethylmethacrylat, Polypropylen, Polystyrol, Polytetrafluorethylen, Polyvinylchlorid, Polyvinylidenchlorid, Pulyurethan, Styrol-Acrylnitril-copolymere, ungesättigte Polyesterharze als Granulate, Pulver oder Gießharz.

Die erfindungsgemäßen Zusammensetzungen können außerdem Stabilisatoren gegen UV- und wettereinflüsse sowie anorganische oder organische Pigmente enthalten, wie oben beschrieben.

Die erfindungsgemäßen Pigmente können einzeln oder in Mischungen in die erfindungsgemäßen Zusammensetzungen eingearbeitet und dort gegebenenfalls zusätzlich durch Scherkräfte auslösende Methoden ausgerichtet werden. Geeignete Methoden zur Ausrichtung der erfindungsgemäßen Pigmente sind Drucken und Rakeln oder, bei magnetischen Pigmenten, Anlegen eines äußeren magnetischen Feldes.

Ein weiterer Gegenstand der vorliegenden Erfindung sind Beschichtungsmittel, die wenigstens ein erfindungsgemäßes Mehrschichtpigment enthalten, vorzugsweise Beschichtungsmittel, die ausgewählt sind unter Effektlacken, -farben oder -folien, insbesondere unter selbstdeckenden Effektlacken, -farben oder -folien.

Ein weiterer Gegenstand der vorliegenden Erfindung ist die Verwendung der erfindungsgemäßen Pigmente im Fahrzeug- und Fahrzeugzubehörsektor, im Freizeit-, Sport- und Spielsektor, im Kosmetikbereich, im Textil-, Leder- oder Schmuckbereich, im Geschenkartikelbereich, in Schreibutensilien, Emballagen oder Brillengestängen, im Bausektor, im Haushaltssektor sowie bei Druckerzeugnissen aller Art, wie beispielsweise Kartonagen, Verpackungen, Tragetaschen, Papiere, Etiketten oder Folien.

Die durch die erfindungsgemäßen cholesterischen Pigmente erzielbaren Farbeffekte umfassen, bedingt durch die Vielfalt der erzielbaren Reflexionswellenlängen, auch den UV- und den IR-Bereich sowie selbstverständlich den Bereich des sichtbaren Lichtes. Werden die erfindungsgemäßen Pigmente auf Banknoten, Scheckkarten, andere bargeldlose Zahlungsmittel oder Ausweise aufgebracht (beispielsweise durch bekannte Druckverfahren) oder in sie eingearbeitet, erschwert dies das identische Kopieren, insbesondere das Fälschen dieser Gegenstände erheblich. Ein weiterer Gegenstand der vorliegenden Erfindung ist somit die Verwendung der erfindungsgemäßen Pigmente zur fälschungserschwerenden Bearbeitung von Gegenständen, insbesondere von Banknoten, Scheckkarten oder anderen bargeldlosen Zahlungsmitteln oder Ausweisen.

Ein weiterer Gegenstand der vorliegenden Erfindung ist die Verwendung der erfindungsgemäßen Zusammensetzungen zur Beschichtung von Gebrauchsgegenständen und zur Lackierung von Fahrzeugen.

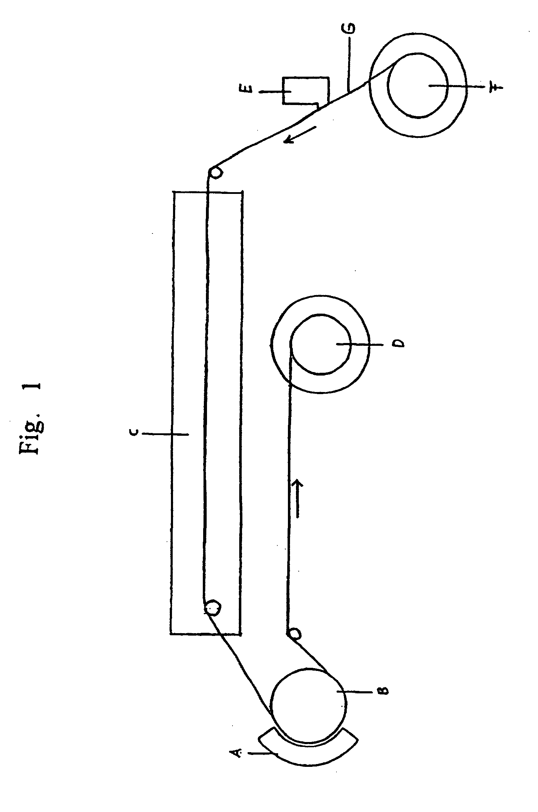

Die Erfindung wird nun anhand der folgenden Ausführungsbeispiele und unter Bezugnahme auf beiliegende Figur 1 näher erläutert. Dabei zeigt

Figur 1 die schematische Darstellung einer erfindungsgemäß einsetzbaren Beschichtungsapparatur

Beispiel 1: Herstellung von Dreischichtpigmenten mit pigmentierter Absorberschicht, umfassend ein cholesterisches strahlungshärtbares Bindemittel

a) Herstellung der 1. Cholesterschicht

Es wurde eine cholesterische Mischung der oben beschriebenen Gruppe b) eingesetzt, die als chirales Monomer eine Verbindung der oben angegebenen Formel 2 und als achirales, nematisches Monomer ein Gemisch von Verbindungen der oben angegebenen Formel 3 enthielt. Das unverdünnte cholesterische Gemisch enthielt 90,5 Gew.-% der achiralen, nematischen Verbindung, 6,5 Gew.-% der chiralen Verbindung und als Photoinitiator 3 Gew.-% 1-Hydrocyclohexylphenylketon, das unter der Bezeichnung Irgacure 184 vertrieben wird. Als Lösungsmittel wurde Methylethylketon verwendet.

Die Beschichtung erfolgte mit einer Beschichtungsapparatur, die in Figur 1 schematisch dargestellt ist. Eine schwarzglänzend, rückseitig vorbeschichtete Polyethylenterephthaltfolie (PET-Folie) (G) mit einer Dicke von 15 μm wurde vom Folienwickel (F) kontinuierlich abgerollt und mit einem Messergießer beschichtet. Die Dicke der cholesterischen Schicht betrug 2 μm. Die Trocknung erfolgte bei 60 °C im Trockner (C). Die Härtung der Schicht erfolgte durch UV-Fixierung in der UV-Anlage (A) während das getrocknete Band über die Kühlwalze (B) geführt wurde. Die gehärtete Cholesterenschicht wurde auf die Rolle (D) aufgewickelt.

Das Reflexionsmaximum der Schicht lag bei 520 nm.

b) Herstellung einer rußpigmentierten Absorberschicht mit cholesterischem Bindemittel

In einem Laborkneter mit einem Nutzvolumen von 300 ml werden 150 g Farbruß Regal 400R (Hersteller: Cabot Corporation) mit 3 g Stearinsäure, 80 g eines phosphonathaltigen Dispergierharzes (50 %-ig in Tetrahydrofuran, beschrieben in DE-A-195 16 784) und 40 g Methylethylketon 1 Stunde geknetet. Die daraus resultierende Knetmasse (Feststoffanteil 70,7 %) wird anschließend in einem Dissolver mit 499 g Methylethylketon auf einen Feststoffgehalt von 25 % eingestellt. Diese Dispersion wird danach in einer Rührwerksmühle (Typ Dispermat SL, Mahlkammervolumen 125 ml, Mahlkörper Zirkonoxid 1-1,25 mm) optimal ausdispergiert. Der Dispergierfortschritt wird dabei mittels einem Interferenzkontrastverfahren (EP-B-0 032 710) verfolgt. Die Endfeinheit ist erreicht, wenn die zu prüfende Oberfläche agglomeratfrei ist. Eine daraus gefertigte Schicht ist hochglänzend und weist eine Grundrauhigkeit von ≤ 100 nm auf. Zu der so erhaltenen Dispersion werden 500 g einer 60 %-igen Cholesterlösung zusammen mit 0,3 g Byk 361 (Byk-Chemie) mittels eines Dissolvers 30 Minuten intensiv eingemischt. Nach Einrühren von 9 g Photoinitiator Irgacure 907 (Ciba Geigy) kann diese Dispersion (Feststoffanteil 39,2 %) aufgetragen werden. Dazu wird sie in einer Schichtdicke von 0,8 μm (Trockendicke) auf die 1. Cholesterschicht analog zu Schritt a) aufgetragen, physikalisch getrocknet und dann unter Stickstoffatmosphäre strahlungsgehärtet.

c) Herstellung der 2. Cholesterschicht

Auf die ausgehärtete Absorberschicht wird analog zu Schritt a) eine weitere Cholesterschicht aufgetragen,

getrocknet und gehärtet. Die 2. Cholesterschicht ist hinsichtlich Zusammensetzung und Schichtdicke mit der 1. Cholesterschicht vergleichbar.

d) Herstellung von Pigmenten

Der ausgehärtete Dreischichtverbund wird von der Trägerfolie entfernt, indem er quer zur Folienbahnrichtung mit einer Rasierklinge verletzt wird und dann mit Druckluft, die durch eine Schlitzdüse gepresst wird, abgeblasen wird. Die beschichtete Folie wird dabei kontinuierlich an der Schlitzdüse vorbeigeführt und der abgeblasene Dreischichtverbund wird in Form von Flocken aufgefangen. Die Dreischichtflocken sind 6 µm dick und zeigen auf beiden Seiten bei senkrechtem Aufblick eine kräftige grüne Farbe mit einem Farbwechsel nach blau, wenn man schräg auf die Flocken schaut.

10 g Cholesterenflocken werden mit 100 g Natriumchlorid gemischt und 6 mal 2 Minuten in einer Schlagmessermühle gemahlen. Nach dem Mahlen wird das Salz mit Wasser herausgewaschen und das Pigment isoliert.

Das auf diese Weise hergestellte Pigment besitzt eine beidseitig hohe Brillanz mit vom Betrachtungswinkel abhängiger Farbigkeit.

Beispiel 2: Herstellung von cholesterischen Dreischichtpigmenten ohne cholesterische Zwischenschicht

a) Herstellung der 1. Cholesterenschicht

Mit Hilfe einer Gießvorrichtung gemäß Beispiel 1 wird eine Lösung bestehend aus 45 Tl. Cholesterenmischung (96,2 % nematische Komponente gemäß Formel (3) und 3,8 % chirale Komponente gemäß Formel (5)), 3 Tl. Photoinitiator Irgacure® 907 (von Fa. Ciba-Geigy), 0,1 Tl. Byk 361 (von Fa. Byk) und 51,9 Tl. Methylethylketon auf eine Polyesterfolie aufgebracht. Die beschichtete Folie wird dann durch einen Trockenkanal geführt, der auf 60°C temperiert ist. Anschließend wird die physikalisch getrocknete Schicht in-line durch Bestrahlen mit UV-Licht unter Stickstoffatmosphäre gehärtet und die beschichtete Folie wird auf eine Spule gewickelt. Die Cholesterenschicht hat eine Dicke von 2 µm und reflektiert senkrecht zur schichtebene Licht mit eimem Maxium der Reflexion bei einer Wellenlänge von 505 nm. Mit dem Auge betrachtet erscheint die Schicht bei senkrechtem Aufblick grün mit einem Farbwechsel nach blau, wenn man schräg auf die Schicht schaut.

b) Herstellung der Zwischenschicht

Die, wie unter a) beschrieben, beschichtete Folie wird mit Hilfe der gleichen Gießvorrichtung mit einer Lösung bestehend aus 70, 82 Tl. schwarzer Druckfarbe Flexoplastol VA/2-Schwarz (von Fa. BASF AG), 7,08 Tl. Reaktivverdünner (Triacrylat von propoxyliertem/ethoxyliertem Trimethylolpropan; Laromer® PO 33F von Fa. BASF AG), 0,85 Tl. Photoinitiator Irgacure® 500 (von Fa. Ciba-Geigy), und 21,25 Tl. Tetrahydrofuran beschichtet. Die beschichtete Folie wird dann durch einen Trockenkanal geführt, der auf 60°C temperiert ist und physikalisch getrocknet. Anschließend wird die Schicht in-line durch Bestrahlen mit UV-Licht unter Stickstoffatmospäre gehärtet und die beschichtete Folie wird auf eine Spule gewickelt. Die schwarze Schicht hat eine Dicke von 1 µm.

c) Herstellung der 2. Cholesterenschicht

Die, wie unter a) und b) beschrieben, beschichtete Folie wird mit Hilfe der gleichen Gießvorrichtung mit einer Lösung bestehend aus 45 Tl. Cholesterenmischung (vgl. Schritt a)), 3 Tl. Photoinitiator Irgacure 907, 0,1 Tl. Byk 361 und 51,9 Tl. Methylethylketon beschichtet. Die beschichtete Folie wird dann durch einen Trokkenkanal geführt, der auf 60°C temperiert ist und physikalisch getrocknet. Anschließend wird die Schicht in-line durch Bestrahlen mit UV-Licht unter Stickstoffatmosphäre gehärtet und die beschichtete Folie wird auf eine Spule gewickelt. Die Cholesterenschicht hat eine Dicke von 2 µm und reflektiert senkrecht zur Schichtebene Licht mit eimem Maxium der Reflexion bei 510 nm. Mit dem Auge betrachtet erscheint die Schicht bei senkrechtem Aufblick grün mit einem Farbwechsel nach blau, wenn man schräg auf die Schicht schaut.

Nach den Arbeitsschritten a), b) und c) erhält man einen 3-Schichtverbund auf der Polyesterträgerfolie. Die mechanische Stabilität des 3-Schichtverbundes wurde durch Messen der Abrißkraft und der Abschälkraft bestimmt. Dazu werden die mit dem 3-Schichtverbund beschichteten Folien in 3,81 mm breite Streifen geschnitten, der Kraftaufnehmer auf den Dreischichtverbund geklebt und die Kraft bestimmt, die zum Einreißen (Abrißkraft) bzw. zum Abziehen (Abschälkraft) der bereits eingerissenen Schicht nötig ist. Die Abrißkraft zwischen der 1. Cholesterenschicht und der schwarzen Zwischenschicht beträgt 0.065 N und die Abschälkraft beträgt 0.005 N.

d) Entfernen des Dreischichtverbundes von der Trägerfolie

Der, wie unter a), b) und c) beschriebene, Dreischichtverbund wird von der Polyesterträgerfolie entfernt, indem der Dreischichtverbund quer zur Folienbahnrichtung mit einer Rasierklinge verletzt wird und dann mit Druckluft, die durch eine Schlitzdüse gepresst wird, abgeblasen wird. Die beschichtete Folie wird dabei kontinuierlich an der Schlitzdüse vorbeigeführt und der abgeblasene Dreischichtverbund wird in Form von Flocken aufgefangen. Die Dreischichtflocken sind 6 µm dick und zeigen auf beiden Seiten bei senkrechtem Aufblick eine kräftige grüne Farbe mit einem Farbwechsel nach blau, wenn man schräg auf die Flocken schaut.

e) Mahlen der Dreischichtflocken zu einem Pigment

10 g Cholesterenflocken, hergestellt wie unter d) beschrieben, werden mit 100 g Natriumchlorid gemischt und 6 mal 2 Minuten in einer Schlagmessermühle gemahlen. Nach dem Mahlen wird das Salz mit Wasser herausgewaschen und das Pigment isoliert. Beim Mahlen kommt es teilweise zu Delamination des Dreischichtpigmentes.

Beispiel 3: Herstellung von cholesterischen Dreischichtpigmenten ohne cholesterische Zwischenschicht

a) Zunächst wird die 1. Cholesterenschicht wie in Beispiel 2 unter a) beschrieben hergestellt.

b) Herstellung der Zwischenschicht

Die, wie unter a) beschrieben, beschichtete Folie wird mit Hilfe der gleichen Gießvorrichtung mit einer Lösung bestehend aus 28,33 Tl. schwarzer Druckfarbe Flexoplastol VA/2-Schwarz (von Fa. BASF AG), 2,83 Tl. Reaktivverdünner (Laromer® PO 33F; von Fa. BASF AG), 0,34 Tl. Photoinitiator Irgacure 500 (von Fa. Ciba-Geigy), 60 Tl. 20 prozentige Lösung eines Copolymers aus Ethylhexylacrylat und Acrylsäure; Acronal® 101 L von Fa. BASF AG) in Tetrahydrofuran und 8,5 Tl. Tetrahydrofuran beschichtet. Die beschichtete Folie wird dann durch einen Trockenkanal geführt, der auf 60°C temperiert ist und physikalisch getrocknet. Anschließend wird die Schicht in-line durch Bestrahlen mit UV-Licht unter Stickstoffatmosphäre gehärtet und die beschichtete Folie wird auf eine Spule gewickelt. Die schwarze Schicht hat eine Dicke von 1 µm.

c) Herstellung der 2. Cholesterenschicht

Auf die schwarze Zwischenschicht wird wie in Beispiel 2 unter c) beschrieben die 2. Cholesterenschicht aufgebracht und dadurch ein Dreischichtverbund erhalten. Die Prüfung der mechanischen Stabilität des Dreischichtverbundes ergibt eine Abrißkraft zwischen der 1. Cholesterenschicht und der schwarzen Zwischenschicht von 0.19 N und eine Abschälkraft von 0.01 N.

d) Entfernen des Dreischichtverbundes von der Trägerfolie

Der Dreischichtverbund wird wie in Beispiel 2 unter d) beschrieben von der Trägerfolie entfernt.

e) Mahlen der Dreischichtflocken zu einem Pigment

Die Mahlung zu einem Pigment erfolgt wie in Beispiel 2 unter e) beschrieben. Die mikroskopische Beurteilung ergibt einen im Vergleich zu Beispiel 2 deutlich geringeren Anteil an delaminierten Pigmentteilchen, die 6 µm dick sind und auf beiden Seiten bei senkrechtem Aufblick eine kräftige grüne Farbe zeigen. Ändert man den Betrachtungswinkel, so erfolgt ein Farbwechsel von grün nach blau. Beim Betrachten auf einem weißen Untergrund sind die Pigmentteilchen deckend.

## Patentansprüche

1. Plättchenförmiges cholesterisches Mehrschichtpigment, **gekennzeichnet durch** die Schichtfolge $A^1/B/A^2$, wobei

$A^1$ und $A^2$ gleich oder verschieden sind und jeweils mindestens eine cholesterische Schicht umfassen, wobei jede cholesterische Schicht eine Schichtdicke im Bereich von 0,5 bis 2 Mikrometer aufweist, und

B für mindestens eine die Schichten $A^1$ und $A^2$ voneinander trennende Zwischenschicht steht, die das die Schichten $A^1$ und $A^2$ transmittierende Licht teilweise oder vollständig absorbiert, wobei B mindestens ein anorganisches oder organisches Absorptionspigment, gegebenenfalls eingebunden in einer Bindemittelmatrix enthält.

**2.** Mehrschichtpigment nach Anspruch 1, wobei $A^1$ und $A^2$ gleiche oder verschiedene optische Eigenschaften besitzen.

**3.** Mehrschichtpigment nach Anspruch 2, wobei $A^1$ und $A^2$ Licht gleicher oder unterschiedlicher Wellenlänge reflektieren und/oder von gleicher oder unterschiedlicher Händigkeit sind.

**4.** Mehrschichtpigment nach einem der vorhergehenden Ansprüche, wobei $A^1$ und $A^2$ cholesterische Gemische, die ausgewählt sind unter

a) mindestens einem cholesterischen, polymerisierbaren Monomer;

b) mindestens einem achiralen, nematischen, polymerisierbaren Monomer und einer chiralen Verbindung;

c) mindestens einem cholesterischen, vernetzbaren Oligimer oder polymer; oder

d) einem cholesterischen Polymer in einem polymerisierbaren Verdünnungsmittel,

e) mindestens einem cholesterischen Polymer, dessen cholesterische Phase durch schnelles Abkühlen unter die Glasübergangstemperatur eingefroren werden kann,

in gehärtetem Zustand enthalten.

**5.** Mehrschichtpigment nach einem der vorhergehenden Ansprüche, wobei B eine Bindemittelmatrix enthält, die wenigstens ein cholesterisches Gemisch gemäß der Definition in Anspruch 4 umfasst.

**6.** Mehrschichtpigment nach Anspruch 5, wobei $A^1$, B und $A^2$ die gleichen cholesterischen Gemische umfassen.

**7.** Verfahren zur Herstellung eines Mehrschichtpigmentes nach einem der vorhergehenden Ansprüche, **dadurch gekennzeichnet, dass** man die Schichten $A^1$, B und $A^2$ gleichzeitig oder zeitlich versetzt übereinander auf einen Träger mittels Gießbeschichtung aufbringt, gleichzeitig oder zeitlich versetzt härtet, die ausgehärteten Schichten vom Träger entfernt und anschließend zu Mehrschichtpigmenten zerkleinert.

**8.** Verfahren nach Anspruch 7, **dadurch gekennzeichnet, dass** man die aufgebrachten Schichten vor der Härtung trocknet.

**9.** Zusammensetzungen enthaltend wenigstens ein Mehrschichtpigment nach einem der Ansprüche 1 bis 6.

**10.** Beschichtungsmittel, enthaltend wenigstens ein Mehrschichtpigment nach einem der Ansprüche 1 bis 6.

**11.** Verwendung von Mehrschichtpigmenten nach einem der Ansprüche 1 bis 6 im Fahrzeug- und Fahrzeugzubehörsektor, im EDV-, Freizeit-, Sport- und Spielsektor, als optische Bauelemente wie Polarisatoren oder Filter, im Kosmetikbereich, im Textil-, Leder- oder Schmuckbereich, im Geschenkartikelbereich, in Schreibutensilien oder auf Brillengestellen, im Bausektor, im Haushaltssektor sowie bei Druckerzeugnissen aller Art sowie zur Herstellung von Farben und Lacken.

**12.** Verwendung von Mehrschichtpigmenten nach einem der Ansprüche 1 bis 6 zur fälschungserschwerenden Bearbeitung von Gegenständen.

**13.** verwendung einer Zusammensetzung nach Anspruch 9 zur Beschichtung von Gebrauchsgegenständen oder zur Lackierung von Fahrzeugen.

## Claims

1. A platelet-shaped cholesteric multilayer pigment which comprises the layer sequence $A^1/B/A^2$ where

| | |
|---|---|
| $A^1$ and $A^2$ | are identical or different and each comprise at least one cholesteric layer, each cholesteric layer having a thickness of from 0.5 to 2 micrometers, and |
| B | is at least one interlayer which separates the layers $A^1$ and $A^2$ from one another and which absorbs all or some of the light transmitted by the layers $A^1$ and $A^2$, where B comprises at least one organic or inorganic absorption pigment, optionally bound in a binder matrix. |

2. A multilayer pigment as claimed in claim 1, where $A^1$ and $A^2$ possess identical or different optical properties.

3. A multilayer pigment as claimed in claim 2, where $A^1$ and $A^2$ reflect light of identical or different wavelength and/or are of identical or different handedness.

4. A multilayer pigment as claimed in any of the preceding claims, where $A^1$ and $A^2$ comprise cholesteric mixtures selected from

   a) at least one cholesteric, polymerizable monomer;

   b) at least one achiral, nematic, polymerizable monomer and one chiral compound;

   c) at least one cholesteric, crosslinkable oligomer or polymer;

   d) a cholesteric polymer in a polymerizable diluent;

   e) at least one cholesteric polymer whose cholesteric phase can be frozen in by rapid cooling to below the glass transition temperature,

   in the cured state.

5. A multilayer pigment as claimed in any of the preceding claims, where B comprises a binder matrix comprising at least one cholesteric mixture as defined in claim 4.

6. A multilayer pigment as claimed in claim 5, where $A^1$, B and $A^2$ comprise the same cholesteric mixtures.

7. A process for producing a multilayer pigment as claimed in any of the preceding claims, which comprises applying the layers $A^1$, B and $A^2$ atop one another to a substrate by means of casting, simultaneously or with a time differential, curing the layers, simultaneously or with a time differential, removing the fully cured layers from the substrate and then comminuting them to give multilayer pigments.

8. A process as claimed in claim 7, wherein the applied layers are dried prior to curing.

9. A composition comprising at least one multilayer pigment as claimed in any of claims 1 to 6.

10. A coating material comprising at least one multilayer pigment as claimed in any of claims 1 to 6.

11. The use of a multilayer pigment as claimed in any of claims 1 to 6 in the vehicle and vehicle accessories sector, in the EDP, leisure, sport and games sector, as an optical component such as a polarizer or filter, in the cosmetics field, in the textile, leather or jewelry field, in the gift product field, in writing utensils or on spectacle frames, in the construction sector, in the domestic sector or in connection with a printed product of any kind, or for preparing an ink or paint.

12. The use of a multilayer pigment as claimed in any of claims 1 to 6 for the anticounterfeiting treatment of an article.

13. The use of a composition as claimed in claim 9 for coating an article of utility or for painting a vehicle.

## Revendications

1. Pigment multicouche cholestérique sous forme de plaquette, **caractérisé par** la série de couches $A^1/B/A^2$, dans lequel

   $A^1$ et $A^2$ sont identiques ou différentes et comprennent chacune au moins une couche cholestérique, chaque couche cholestérique présentant une épaisseur de couche allant de 0,5 à 2 micromètres,

   B représente au moins une couche intermédiaire séparant les couches $A^1$ et $A^2$ l'une de l'autre, qui absorbe partiellement ou totalement la lumière transmise par les couches $A^1$ et $A^2$, B contenant au moins un pigment d'absorption inorganique ou organique, éventuellement intégré dans une matrice de liant.

2. Pigment multicouche selon la revendication 1, dans lequel $A^1$ et $A^2$ possèdent des propriétés optiques identiques ou différentes.

3. Pigment multicouche selon la revendication 2, dans lequel $A^1$ et $A^2$ reflètent la lumière de longueurs d'ondes identiques ou différentes et/ou sont de chiralité identique ou différente.

4. Pigment multicouche selon l'une quelconque des revendications précédentes, dans lequel $A^1$ et $A^2$ contiennent des mélanges cholestériques choisis parmi

   a) au moins un monomère cholestérique polymérisable ;
   b) au moins un monomère achiral, nématique polymérisable et un composé chiral ;
   c) au moins un oligomère ou polymère cholestérique réticulable ;
   d) un polymère cholestérique dans un diluant polymérisable,
   e) au moins un polymère cholestérique dont la phase cholestérique peut être figée au moyen d'un refroidissement rapide en dessous de la température de transition vitreuse,

   à l'état durci.

5. Pigment multicouche selon l'une quelconque des revendications précédentes, dans lequel B contient une matrice de liant qui comprend au moins un mélange cholestérique selon la définition dans la revendication 4.

6. Pigment multicouche selon la revendication 5, dans lequel $A^1$, B et $A^2$ comprennent les mêmes mélanges cholestériques.

7. Procédé de préparation d'un pigment multicouche selon l'une quelconque des revendications précédentes, **caractérisé en ce que** l'on dépose, au moyen d'une coulée, simultanément ou successivement, les couches $A^1$, B et $A^2$ les unes sur les autres sur un support, on retire les couches durcies du support puis on les broie en pigments multicouches.

8. Procédé selon la revendication 7, **caractérisé en ce que** l'on sèche les couches déposées avant le durcissement.

9. Compositions contenant au moins un pigment multicouche selon l'une quelconque des revendications 1 à 6.

10. Agent de revêtement, contenant au moins un pigment multicouche selon l'une quelconque des revendications 1 à 6.

11. Utilisation de pigments multicouche selon l'une quelconque des revendications 1 à 6 dans le domaine de l'automobile et des accessoires automobiles, dans le domaine de l'informatique, des loisirs, du sport et des jeux, en tant que composants optiques tels que des polariseurs ou des filtres, dans le domaine des cosmétiques, dans le domaine du textile, du cuir ou des bijoux, dans le domaine des articles de cadeaux, dans les articles de papeterie ou sur les montures de lunettes, dans le domaine de la construction, dans le domaine domestique ainsi que pour les imprimés de tout type et pour la préparation de peintures et de vernis.

12. Utilisation de pigments multicouche selon l'une quelconque des revendications 1 à 6 pour le traitement d'objets destiné à les rendre infalsifiables.

13. Utilisation d'une composition selon la revendication 9 pour le revêtement d'objets usuels ou pour le laquage d'automobiles.

Fig. 1